(19) Europäisches Patentamt / European Patent Office / Office européen des brevets

(11) EP 3 992 695 A1

(12) EUROPEAN PATENT APPLICATION
published in accordance with Art. 153(4) EPC

(43) Date of publication:
04.05.2022 Bulletin 2022/18

(21) Application number: 19934953.1

(22) Date of filing: 27.06.2019

(51) International Patent Classification (IPC):
*G02C 7/04* (2006.01) *A61F 2/16* (2006.01)
*G02B 1/04* (2006.01) *G02B 5/23* (2006.01)

(52) Cooperative Patent Classification (CPC):
A61F 2/16; G02B 1/04; G02B 5/23; G02C 7/04

(86) International application number:
PCT/JP2019/025687

(87) International publication number:
WO 2020/261502 (30.12.2020 Gazette 2020/53)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Menicon Co., Ltd.
Nagoya-shi, Aichi 460-0006 (JP)
- Tokuyama Corporation
Shunan-shi, Yamaguchi 745-8648 (JP)

(72) Inventors:
- TOMIDA, Tomomi
Kasugai-shi, Aichi 487-0032 (JP)
- NAKADA, Kazuhiko
Kasugai-shi, Aichi 487-0032 (JP)
- BABA, Masaki
Kasugai-shi, Aichi 487-0032 (JP)
- NIIDA, Satomu
Kasugai-shi, Aichi 487-0032 (JP)
- MOMODA, Junji
Shunan-shi, Yamaguchi 745-8648 (JP)
- MIYAZAKI, Masayuki
Shunan-shi, Yamaguchi 745-8648 (JP)
- TAKENAKA, Junji
Shunan-shi, Yamaguchi 745-8648 (JP)

(74) Representative: Winter, Brandl - Partnerschaft mbB
Alois-Steinecker-Straße 22
85354 Freising (DE)

# (54) OPHTHALMIC MEDICAL INSTRUMENT INCLUDING PHOTOCHROMIC POLYMER AND PRODUCTION METHOD FOR OPHTHALMIC MEDICAL INSTRUMENT

(57) Provided is an ophthalmic medical device including a photochromic polymer having higher transparency under an inactive state through use of a photochromic monomer. An ophthalmic medical device according to one embodiment of the present invention includes a photochromic polymer obtained by polymerizing monomer components including a photochromic monomer and a lactam ring-containing monomer, wherein a content ratio of the lactam ring-containing monomer in the monomer components is from 10 wt% to 50 wt%. The ophthalmic medical device is typically a contact lens or an intraocular lens.

EP 3 992 695 A1

## Description

### Technical Field

The present invention relates to an ophthalmic medical device including a photochromic polymer and a method of producing the device.

### Background Art

A photochromic compound has at least two states having the same molecular weight and different absorption spectra. For example, the compound can switch from a first state to a second state through irradiation with light, and can return to the first state in response to the blocking of the light or thermal energy, or in response to irradiation with light having any other wavelength. For example, the following pair of photochromic glasses obtained by blending a glass lens with the photochromic compound has been commercially available. The pair of photochromic glasses can be used as a pair of normal glasses in a room where a UV irradiation dose is small, and can be used as a pair of sunglasses in the open air where the UV irradiation dose is large through use of the property of the photochromic compound by which the compound can be reversibly isomerized between the at least two states having different absorption spectra as described above.

Meanwhile, with regard to a contact lens, there have been proposed a photochromic contact lens obtained by mixing a nonpolymerizable photochromic compound in a polymer lens (e.g., Patent Literature 1) and a photochromic contact lens formed from a polymer obtained by polymerizing monomer components including a polymerizable photochromic compound (e.g., Patent Literature 2) .

### Citation List

### Patent Literature

[PTL 1] WO 2004/068215 A1
[PTL 2] JP 2017-49615 A

### Summary of Invention

### Technical Problem

When a photochromic property is imparted to a contact lens or an intraocular lens, a polymerizable photochromic compound (hereinafter referred to as "photochromic monomer") is preferably used from the viewpoints of safety and stability in a wet environment, such as the top of a cornea or the interior of an eye, and the lens desirably has high transparency under an inactive state. Meanwhile, the photochromic monomer has insufficient compatibility with any other monomer, and hence the transparency of a lens to be obtained may be impaired. In Patent Literature 2, a uniform reactive mixture is obtained by adding an organic solvent at the time of the mixing of the monomer components including the photochromic monomer, but the transparency of the polymer to be obtained is susceptible to improvement. In addition, the use of the organic solvent may lead to frequent occurrence of chain transfer during the polymerization to make the photochromic polymer to be obtained brittle. Further, a large amount of a water-soluble organic solvent or water is required in a step of removing the organic solvent from the photochromic polymer, and hence the use of the organic solvent is not preferred from the viewpoint of environmental protection.

The present invention has been made to solve the above-mentioned problems, and a primary object of the present invention is to provide an ophthalmic medical device including a photochromic polymer having higher transparency under an inactive state through use of a photochromic monomer. A further object of the present invention is to suppress or avoid the use of an organic solvent at the time of the production of the device.

### Solution to Problem

According to one embodiment of the present invention, there is provided an ophthalmic medical device, including a photochromic polymer obtained by polymerizing monomer components including a photochromic monomer and a lactam ring-containing monomer, wherein a content ratio of the lactam ring-containing monomer in the monomer components is from 10 wt% to 50 wt%, and wherein the ophthalmic medical device is a contact lens or an intraocular lens.

In one embodiment, the lactam ring-containing monomer includes at least one kind selected from an N-vinyl lactam and a methylene lactam.

In one embodiment, the monomer components further include at least one kind selected from an N,N-dialkyl (meth)acrylamide and an N,N-dialkylaminoalkyl (meth)acrylamide.

In one embodiment, a total content ratio of the lactam ring-containing monomer, and the at least one kind selected from the N,N-dialkyl (meth)acrylamide and the N,N-dialkylaminoalkyl (meth)acrylamide in the monomer components is from 25 wt% to 65 wt%.

In one embodiment, the monomer components further include a silicone-containing monomer.

In one embodiment, a content ratio of the photochromic monomer in the monomer components is from 0.001 wt% to 5 wt%.

In one embodiment, the photochromic monomer is a T-type photochromic compound.

In one embodiment, the photochromic monomer is a naphthopyran compound.

In one embodiment, the photochromic monomer is represented by the following structural formula (1):

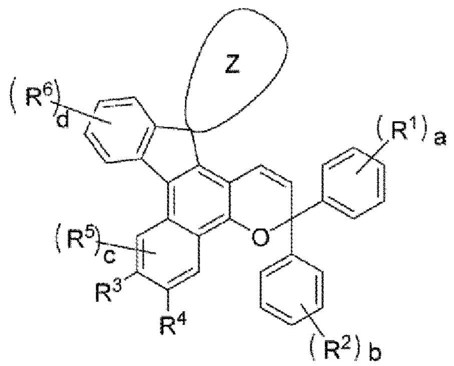

(1)

in the formula (1), $R^1$ to $R^6$, "a" to "d", and a ring Z are described later.

In one embodiment, the monomer components further include at least one kind selected from an alkyl (meth)acrylate, an alkoxyalkyl (meth)acrylate, and an aromatic ring-containing (meth)acrylate.

In one embodiment, a content ratio of a hydroxyl group-containing monomer in the monomer components is 20 wt% or less.

According to another embodiment of the present invention, there is provided a method of producing the ophthalmic medical device, including polymerizing a reactive mixture, which contains monomer components including a photochromic monomer and a lactam ring-containing monomer, and is free of an organic solvent or contains the organic solvent at a content ratio of 10 parts by weight or less with respect to 100 parts by weight of the monomer components, to provide a photochromic polymer, wherein a content ratio of the lactam ring-containing monomer in the monomer components is from 10 wt% to 50 wt%.

In one embodiment, a content ratio of a hydroxyl group-containing monomer in the monomer components is 20 wt% or less.

In one embodiment, the polymerizing includes thermal polymerization and photopolymerization.

According to still another embodiment of the present invention, there is provided an ophthalmic medical device, including a photochromic polymer, which has a repeating unit derived from a photochromic monomer and is capable of changing from an inactive state to an activated state having a visible light transmittance lower than that of the inactive state through absorption of light energy, wherein the ophthalmic medical device has a spectral transmittance of more than 90% in at least part of wavelengths in a wavelength region of less than 700 nm under the inactive state, and wherein the ophthalmic medical device is a contact lens or an intraocular lens.

In one embodiment, the ophthalmic medical device has a spectral transmittance of less than 70% in at least part of wavelengths in a visible light region under the activated state.

In one embodiment, the ophthalmic medical device has a spectral transmittance of less than 70% in at least part of wavelengths in a wavelength region of from 500 nm to 700 nm under the activated state.

In one embodiment, the ophthalmic medical device has a spectral transmittance of less than 70% over an entirety of a wavelength region of from 530 nm to 670 nm under the activated state.

In one embodiment, the ophthalmic medical device has a spectral transmittance of 15% or more and less than 70% in at least part of wavelengths in a wavelength region of from 500 nm to 700 nm under the activated state.

In one embodiment, the ophthalmic medical device has a spectral transmittance of 15% or more and less than 70% over an entirety of a wavelength region of from 530 nm to 670 nm under the activated state.

In one embodiment, the ophthalmic medical device has a spectral transmittance of 80% or less in a blue light

region under the activated state.

In one embodiment, the ophthalmic medical device has a spectral transmittance of 70% or less in a blue light region under the activated state.

In one embodiment, the ophthalmic medical device has a luminous transmittance of 75% or more in a wavelength region of from 380 nm to 780 nm under the inactive state.

In one embodiment, the ophthalmic medical device is a hydrogel lens having a Dk value of from 25 barrer to 160 barrer, having a water content of from 25 wt% to 80 wt%, and having a Young's modulus of from 0.2 MPa to 2.0 MPa.

In one embodiment, the ophthalmic medical device is an oxygen-permeable hard contact lens having a Dk value of from 130 barrer to 250 barrer and having a water absorption ratio of 1.0 wt% or less.

In one embodiment, the ophthalmic medical device is an oxygen-permeable soft contact lens having a Dk value of from 50 barrer to 200 barrer and having a shape recovery ratio of 25% or less.

In one embodiment, the ophthalmic medical device is an intraocular lens having an elongation of from 170% to 600% and having a water absorption ratio of from 1.5 wt% to 4.5 wt%.

In one embodiment, the ophthalmic medical device changes from the inactive state to the activated state within 1 minute from a start of irradiation with light having an illuminance of 50,000 lux from a xenon lamp.

Advantageous Effects of Invention

In the present invention, the lactam ring-containing monomer is used at a specific blending ratio as a monomer copolymerizable with the photochromic monomer. Thus, the compatibility of the photochromic monomer in the monomer components can be secured, and hence the photochromic polymer having excellent transparency under its inactive state (consequently, the ophthalmic medical device having excellent transparency under its inactive state) can be obtained without reliance on the use of the organic solvent.

Brief Description of Drawings

FIGS. 1 are light transmittance spectra of contact lenses obtained in Experimental Examples.

FIG. 2 is light transmittance spectra of the contact lens obtained in Experimental Example before and after its extraction treatment.

FIG. 3 is light transmittance spectra of the contact lens obtained in Experimental Example before and after its extraction treatment.

FIG. 4 is photographs of the contact lenses obtained in Experimental Examples.

Description of Embodiments

Preferred embodiments of the present invention are described below. However, the present invention is not limited to these embodiments.

A. Definitions of Terms

The definitions of terms as used herein are as described below.

(1) The term "activated state" means that a photochromic monomer (specifically, a constituent unit derived from the photochromic monomer) in a photochromic polymer is in a second state by switching from a first state due to absorption of energy provided by light irradiation, and the term "inactive state" means that the photochromic monomer in the polymer material is in the first state by switching from the second state. However, a change at the time of the activation of the photochromic compound from the inactive state or at the time of the inactivation thereof from the activated state is generally continuous. Accordingly, a state in which no further change in transmittance of the compound occurs even when irradiation with light having a predetermined wavelength is further continued can be defined as the "activated state," and a state in which no further change in transmittance thereof occurs even when a state in which the irradiation with the light having the predetermined wavelength is absent is further continued can be defined as the "inactive state." An ophthalmic medical device of the present invention is typically in the decolored state of showing a higher visible light transmittance under the inactive state than under the activated state, and is

brought into the activated state (color developed (colored) state) having a low visible light transmittance by the absorption of light energy. The ratio of the "degree of color development (coloring)" in the "activated state" to the "degree of color development (coloring)" in the "inactive state" may hereinafter be simply referred to as "color development density" (a high "color development density" means that the color of the device is dark).

(2) The terms "luminous transmittance," "spectral transmittance," and "visible light transmittance" refer to values determined in conformity with the specifications of JIS T 7333:2018 (ISO 8980-3:2013).

(3) The term "visible light region" means a wavelength region of from 380 nm to 780 nm.

(4) The term "blue light region" means a wavelength region of from 380 nm to 500 nm.

(5) The term "(meth)acrylic" means methacrylic and/or acrylic.

B. Ophthalmic Medical Device

The ophthalmic medical device according to the embodiment of the present invention is typically a contact lens or an intraocular lens. The ophthalmic medical device includes a photochromic polymer obtained by polymerizing monomer components including a photochromic monomer and a lactam ring-containing monomer. The blending ratio of the lactam ring-containing monomer in the monomer components is from 10 wt% to 50 wt%. When the lactam ring-containing monomer is used at such content ratio in combination with the photochromic monomer, a photochromic polymer having excellent transparency under its inactive state (consequently, an ophthalmic medical device having excellent transparency under its inactive state) can be obtained.

The monomer components may further include any appropriate monomer in accordance with, for example, physical properties that the ophthalmic medical device is desired to have and its applications. The monomer components may include, for example, a silicone-containing monomer, a hydrophilic monomer, or a cross-linkable monomer in addition to the photochromic monomer and the lactam ring-containing monomer.

B-1. Photochromic Monomer

As a matter of course, the photochromic monomer to be used in the present invention is a compound having both of a moiety that exhibits a photochromic characteristic and a polymerizable group. The photochromic monomer may be any one of: a T type in which the monomer switches from an inactive state to an activated state through irradiation with light having a predetermined wavelength $\lambda$, and then returns to the inactive state in response to the blocking of the light or thermal energy; and a P type in which the monomer returns to the inactive state in response to irradiation with light having any other wavelength $\lambda'$ after the switching. Of those, a T-type photochromic monomer is preferably used. An ophthalmic medical device using the T-type photochromic monomer is brought into an activated state (colored state) in, for example, the open air where a UV irradiation dose is large, and can return to an inactive state (decolored state) in a room where the UV irradiation dose is small.

The wavelength $\lambda$ at which the photochromic monomer is activated may fall within the range of, for example, from 200 nm to 500 nm, preferably from 200 nm to 480 nm, more preferably from 200 nm to 450 nm.

Examples of the P-type photochromic monomer include a fulgide compound, a diarylethene compound, and a phenoxynaphthacenequinone compound. In addition, examples of the T-type photochromic monomer include an azobenzene compound, a hexaarylbiimidazole compound, a spiropyran-spirooxazine compound, a naphthopyran compound, an anthracene dimer, and a salicylideneaniline compound. Of those, a naphthopyran compound is preferred. The photochromic monomers may be used alone or in combination thereof.

The heat return reaction of the T-type photochromic monomer is preferably fast. An ophthalmic medical device using such photochromic monomer can return to an inactive state preferably within 60 seconds, more preferably within 30 seconds after the completion of the irradiation with the light having the predetermined wavelength under a cornea temperature (e.g., 35°C$\pm$2°C) .

A naphthopyran-based photochromic monomer to be preferably used in the present invention has a 3,3-diphenylindenonaphthopyran structure represented by the following structural formula (1) as a basic skeleton (hereinafter sometimes simply referred to as "indenonaphthopyran skeleton").

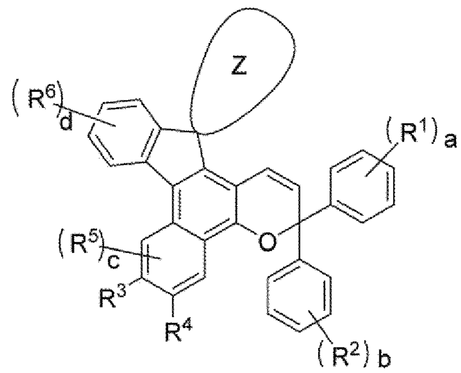

(1)

In general, a photochromic monomer having an indenonaphthopyran structure is known to show an excellent photochromic characteristic. Specific substituents are hereinafter described in due order.

<$R^1$ and $R^2$>

Both of $R^1$ and $R^2$ represent substituents substituting phenyl groups bonded to a carbon atom at the 3-position of the indenonaphthopyran skeleton.

$R^1$ and $R^2$ each independently represent a group having a radical-polymerizable group, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, or an arylthio group having 6 to 10 carbon atoms that may have a substituent.

"a" represents an integer of from 0 to 5, and "b" represents an integer of from 0 to 5, provided that a+b=1 to 10, at least one of $R^1$ or $R^2$ represents the group having the radical-polymerizable group. As a matter of course, as long as at least one of $R^1$ or $R^2$ represents the radical-polymerizable group, when a plurality of $R^1$s exist, $R^1$s may represent groups identical to or different from each other. In addition, the same holds true for $R^2$: when a plurality of $R^2$s exist, $R^2$s may represent groups identical to or different from each other.

Suitable examples of the alkyl group having 1 to 6 carbon atoms may include a methyl group, an ethyl group, a n-propyl group, an isopropyl group, a n-butyl group, a sec-butyl group, a tert-butyl group, a pentyl group, and a hexyl group.

The haloalkyl group having 1 to 6 carbon atoms is preferably an alkyl group substituted with a fluorine atom, a chlorine atom, or a bromine atom. Suitable examples of the haloalkyl group may include a trifluoromethyl group, a tetrafluoroethyl group, a chloromethyl group, a 2-chloroethyl group, and a bromomethyl group.

Examples of the cycloalkyl group having 3 to 8 carbon atoms may include a cyclopropyl group, a cyclobutyl group, a cyclopentyl group, and a cyclohexyl group.

Suitable examples of the alkoxy group having 1 to 6 carbon atoms may include a methoxy group, an ethoxy group, a n-propoxy group, an isopropoxy group, a n-butoxy group, a sec-butoxy group, and a tert-butoxy group.

The amino group is not limited to a primary amino group ($-NH_2$), and may be a secondary or tertiary amino group obtained by substituting one or two hydrogen atoms of the primary amino group. Examples of the substituent of such amino group include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, and a heteroaryl group having 4 to 14 carbon atoms. Suitable examples of the amino group may include an amino group, a methylamino group, a dimethylamino group, an ethylamino group, a diethylamino group, a phenylamino group, and a diphenylamino group.

Preferred examples of the heterocyclic group may include: aliphatic heterocyclic groups, such as a morpholino group, a piperidino group, a pyrrolidinyl group, a piperazino group, and an N-methylpiperazino group; and aromatic heterocyclic groups, such as an indolinyl group. Further, the heterocyclic group may have a substituent. The substituent is preferably, for example, an alkyl group. Suitable examples of the heterocyclic group having a substituent include a 2,6-dimethylmorpholino group, a 2,6-dimethylpiperidino group, and a 2,2,6,6-tetramethylpiperidino group.

Examples of the halogen atom may include a fluorine atom, a chlorine atom, a bromine atom, and an iodine atom.

Examples of the alkylthio group having 1 to 6 carbon atoms may include a methylthio group, an ethylthio group, a n-propylthio group, an isopropylthio group, a n-butylthio group, a sec-butylthio group, and a t-butylthio group.

Examples of the arylthio group having 6 to 10 carbon atoms may include a phenylthio group, a 1-naphthylthio group, and a 2-naphthylthio group.

One to five hydrogen atoms, particularly preferably one to four hydrogen atoms of the aromatic ring of the

arylthio group may each be substituted with an alkyl group having 1 to 6 carbon atoms, an alkoxy group having 1 to 6 carbon atoms, a cycloalkyl group having 3 to 8 carbon atoms, or a halogen atom. The number of carbon atoms of a substituent is not included in the number of carbon atoms of the arylthio group, that is, from 6 to 10.

From the viewpoint of the exhibition of an excellent photochromic characteristic, $R^1$ and $R^2$ each preferably represent a group selected from the alkyl group, the alkoxy group, the amino group, the substituted amino group, the heterocyclic group, and the halogen atom out of the foregoing groups as a group except the group having the radical-polymerizable group. Particularly suitable examples thereof include a methyl group, a methoxy group, a dimethylamino group, a morpholino group, a piperidino group, and a fluoro group.

As a particularly preferred group, the number of $R^1$s and $R^2$s each representing a group except the group having the radical-polymerizable group is preferably 1 or 0. In other words, each of the phenyl groups of the photochromic monomer is preferably in the state of being substituted with one group except the group having the radical-polymerizable group, or with no such group. When the phenyl group is substituted with one group, the phenyl group is preferably substituted with the group at its para position.

<$R^1$ and $R^2$; Group having Radical-polymerizable Group>

The group having the radical-polymerizable group is a group represented by the following formula (2).

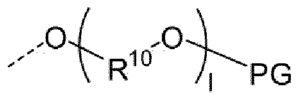

(2)

In the formula (2), $R^{10}$ represents a linear or branched alkylene group having 1 to 10 carbon atoms. Of those, an alkylene group having 1 to 5 carbon atoms is preferred. Suitable examples of the alkylene group having 1 to 5 carbon atoms may include a methylene group, an ethylene group, a n-propylene group, an isopropylene group, a n-butylene group, a sec-butylene group, a tert-butylene group, and a pentylene group.

"l" represents an integer of from 0 to 50. In consideration of the productivity of the photochromic monomer itself and an effect exhibited by the monomer, "l" represents preferably an integer of from 1 to 20, more preferably an integer of from 1 to 10, still more preferably an integer of from 1 to 5, most preferably 1. "l" represents the number of repeating units (-$R^{10}$O-). When "l" represents 2 or more, the unit groups ((-$R^{10}$O-) groups) in "l" pairs of parentheses may be groups identical to or different from each other.

PG represents the radical-polymerizable group, and examples thereof include a vinyl group, a 1-chlorovinyl group, an allyl group, a styryl group, a (meth)acrylic group, a 2-(methacryloxy)ethylcarbamyl group, a 2-(methacryloxy)ethoxycarbonyl group, and a crotyl group. Of those, a (meth)acrylic group is most preferred in consideration of the productivity of the photochromic monomer itself and the performance of an ophthalmic medical device.

<Numbers and Substitution Positions of $R^1$s and $R^2$s>

In the formula (1), "a" represents the number (substitution number) of $R^1$s and "b" represents the number (substitution number) of $R^2$s. In addition, "a" represents an integer of from 0 to 5, and "b" represents an integer of from 0 to 5, provided that a+b=1 to 10, and at least one of $R^1$ or $R^2$ represents the group having the radical-polymerizable group. In other words, as long as at least one of $R^1$ or $R^2$ represents the group having the radical-polymerizable group, the other symbols may each represent a group except the group having the radical-polymerizable group described in the section <$R^1$ and $R^2$> or a hydrogen atom.

The most preferred aspect out of those aspects is a case in which the number of the group having the radical-polymerizable group is 1. When the number of the group having the radical-polymerizable group is 2 or more, the photochromic monomer tends to be cross-linked to reduce its photochromic characteristic.

In addition, a position substituted with the group having the radical-polymerizable group is preferably the para position of each of the phenyl groups of 3,3-diphenylindenonaphthopyran. Accordingly, a preferred aspect is a case in which the para position of one of the phenyl groups is substituted with the group having the radical-polymerizable group. In this case, the other phenyl group is not limited, but of course, a case in which one substituent is present at its para position or no substituent is present thereat (a hydrogen atom is also present at the para position) is preferred. Of such cases, a case in which the other phenyl group is free of any substituent, or is substituted with the alkyl group, the alkoxy group, or the heterocyclic group at its para position is preferred.

<Ring Z (Group)>

A ring Z(cyclic group being spiro-bonded to the carbon atom at the 13-position) represented by the following formula (Z), the ring being spiro-bonded to a carbon atom at a 13-position of the indenonaphthopyran skeleton:

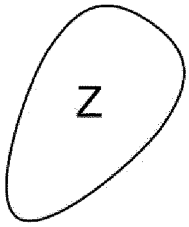

(Z)

is

an aliphatic cyclic group that may have a substituent, the group having 3 to 20 carbon atoms for forming the ring together with the carbon atom at the 13-position,
a condensed polycyclic group obtained by condensing the aliphatic cyclic group with an aromatic ring or an aromatic heterocycle that may have a substituent,
a heterocyclic group that may have a substituent, the group having 3 to 20 atoms for forming the ring together with the carbon atom at the 13-position, or
a condensed polycyclic group obtained by condensing the heterocyclic group with an aromatic ring or an aromatic heterocycle that may have a substituent. As a matter of course, the number of carbon atoms or the number of atoms described in each of the cyclic groups represents the number of carbon atoms, or atoms, for forming the ring, and does not include the number of carbon atoms, or atoms, of a substituent.

Examples of the aliphatic cyclic group include a cyclopentane ring group, a cyclohexane ring group, a cyclooctane ring group, a cycloheptane ring group, a norbornane ring group, a bicyclononane ring group, and an adamantane ring group.

In addition, an example of the condensed polycyclic group obtained by condensing the aliphatic cyclic group with an aromatic ring or an aromatic heterocycle is a phenanthrene ring group.

Examples of the heterocyclic group include a thiophene ring group, a furan ring group, and a pyridine ring group.

In addition, examples of the condensed polycyclic group obtained by condensing the heterocyclic group with an aromatic ring or an aromatic heterocycle include a phenylfuran ring group and a biphenylthiophene ring group.

The aliphatic cyclic group, the condensed polycyclic group obtained by condensing the aliphatic cyclic group with an aromatic ring or an aromatic heterocycle, the heterocyclic group, or the condensed polycyclic group obtained by condensing the heterocyclic group with an aromatic ring or an aromatic heterocycle may have a substituent. An example of the substituent substituting each of the cyclic groups (condensed polycyclic groups) is at least one kind of substituent selected from the group consisting of: an alkyl group having 1 to 6 carbon atoms; a haloalkyl group having 1 to 6 carbon atoms; a cycloalkyl group having 3 to 8 carbon atoms; an alkoxy group having 1 to 6 carbon atoms; an amino group; a substituted amino group; and a halogen atom. Examples of the alkyl group, the haloalkyl group, the cycloalkyl group, the alkoxy group, the amino group, the substituted amino group, and the halogen atom include the same groups as the groups that have already been described in the section <$R^1$ and $R^2$>. Of the substituents of the ring Z, the alkyl group having 1 to 6 carbon atoms, the cycloalkyl group, the haloalkyl group having 1 to 6 carbon atoms, or the alkoxy group having 1 to 6 carbon atoms is particularly preferred as a substituent causing the photochromic monomer to exhibit a particularly excellent effect.

Of the rings Z, to obtain a high color development density while securing a fast color fading rate, the aliphatic cyclic group having 5 to 16 carbon atoms for forming the ring, a cyclic group obtained by substituting such aliphatic cyclic group with an alkyl group having 1 to 6 carbon atoms (preferably an alkyl group having 1 to 3 carbon atoms), or a cyclic group obtained by bonding or condensing the aliphatic cyclic group with a cycloalkyl group having 3 to 8 carbon atoms is preferred.

A particularly suitable specific example of the ring Z is an unsubstituted cyclohexane ring group, cycloheptane ring group, cyclooctane ring group, cyclononane ring group, cyclodecane ring group, cycloundecane ring group, or cyclododecane ring group, each of which is free of any substituent.

In addition, the ring Z may be a cyclohexane ring group, but when the ring is the cyclohexane ring group, the cyclohexane ring group is substituted with preferably an alkyl group having 1 to 3 carbon atoms, more preferably an alkyl group having 1 or 2 carbon atoms. Further, in the case of the cyclohexane ring group substituted with the alkyl

group, the substitution number of the alkyl groups is preferably from 1 to 10, more preferably from 2 to 6.

Further, in order that the effect through which the photochromic monomer has a high color development density while having a fast color fading rate may be significant, the ring Z is preferably represented by any one of the following formulae.

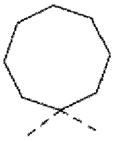 , 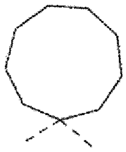 , 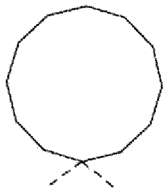 , 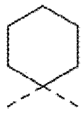 ,

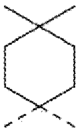 , 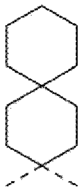 , 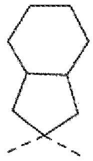 , 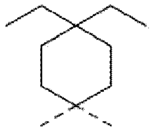 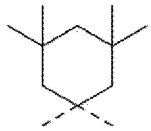

In each of the formulae, a carbon atom having bonding hands represented by dotted lines is the carbon atom at the 13-position.

In addition, in order that in particular, the photochromic monomer can more significantly exhibit such effect that a high color development density is achieved, out of the cyclic groups, a group having 6 to 15 carbon atoms for forming the ring together with the carbon atom at the 13-position is preferred, and a group having 7 to 12 carbon atoms for forming the ring together with the carbon atom at the 13-position is more preferred.

Of the preferred cyclic groups, the cyclic group to be adopted for a case in which a photochromic monomer having a higher color development density is produced and the cyclic group to be adopted for a case in which a photochromic monomer having a faster color fading rate is produced are preferably different from each other.

That is, to obtain a higher color development density, a cyclic group represented by any one of the following formulae is preferably adopted.

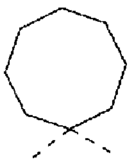 , 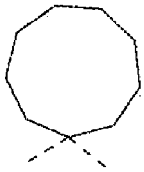 , 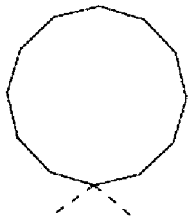 , 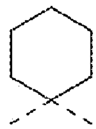 ,

When such a cycloalkane ring having 6 to 12 carbon atoms (containing the carbon atom at the 13-position) as described above, the ring being free of any substituent, is adopted, a photochromic monomer having a particularly high color development density is obtained.

Meanwhile, to obtain a photochromic monomer capable of responding at a higher speed (having a faster color fading rate), a cyclic group represented by any one of the following formulae is preferably adopted.

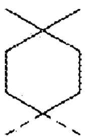 , 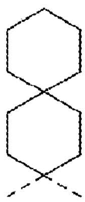 , 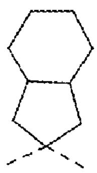 , 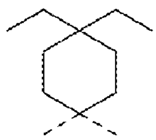 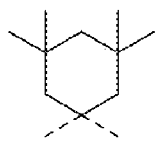

The adoption of such a group as described above can provide a photochromic monomer capable of responding at a higher speed.

<$R^3$>

$R^3$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a cyano group, a halogen atom, an alkylthio group having 1 to 6 carbon atoms, an arylthio group having 6 to 10 carbon atoms that may have a substituent, a nitro group, a formyl group, a hydroxycarbonyl group, an alkylcarbonyl group having 2 to 7 carbon atoms, an alkoxycarbonyl group having 2 to 7 carbon atoms, an aralkyl group having 7 to 11 carbon atoms that may have a substituent, an aralkoxy group having 7 to 11 carbon atoms that may have a substituent, an aryloxy group having 6 to 12 carbon atoms that may have a substituent, an aryl group having 6 to 12 carbon atoms that may have a substituent, a heteroaryl group having 3 to 12 carbon atoms that may have a substituent, a thiol group, an alkoxyalkylthio group having 2 to 9 carbon atoms, a haloalkylthio group having 1 to 6 carbon atoms, or a cycloalkylthio group having 3 to 8 carbon atoms.

Examples of the alkyl group having 1 to 6 carbon atoms, the haloalkyl group having 1 to 6 carbon atoms, the cycloalkyl group having 3 to 8 carbon atoms, the alkoxy group having 1 to 6 carbon atoms, the halogen atom, the alkylthio group having 1 to 6 carbon atoms, or the arylthio group having 6 to 10 carbon atoms that may have a substituent out of the foregoing groups include the same groups as the groups described in the section <$R^1$ and $R^2$>, and preferred groups thereof are also the same.

Examples of the alkylcarbonyl group having 2 to 7 carbon atoms include an acetyl group and an ethylcarbonyl group.

Examples of the alkoxycarbonyl group having 2 to 7 carbon atoms include a methoxycarbonyl group and an ethoxycarbonyl group.

Examples of the aralkyl group having 7 to 11 carbon atoms may include a benzyl group, a phenylethyl group, a phenylpropyl group, a phenylbutyl group, and a naphthylmethyl group.

Examples of the aralkoxy group having 7 to 11 carbon atoms may include a benzyloxy group and a naphthylmethoxy group.

Examples of the aryloxy group having 6 to 12 carbon atoms may include a phenyloxy group and a naphthyloxy group.

Examples of the aryl group having 6 to 12 carbon atoms may include a phenyl group, a 1-naphthyl group, and a 2-naphthyl group.

Examples of the heteroaryl group having 3 to 12 carbon atoms may include a thienyl group, a furyl group, a pyrrolinyl group, a pyridyl group, a benzothienyl group, a benzofuranyl group, and a benzopyrrolinyl group.

Examples of the alkoxyalkylthio group having 2 to 9 carbon atoms may include a methoxymethylthio group, a methoxyethylthio group, a methoxy-n-propylthio group, a methoxy-n-butylthio group, an ethoxyethylthio group, and a n-propoxypropylthio group.

Examples of the haloalkylthio group having 1 to 6 carbon atoms may include a trifluoromethylthio group, a tetrafluoroethylthio group, a chloromethylthio group, a 2-chloroethylthio group, and a bromomethylthio group.

Examples of the cycloalkylthio group having 3 to 8 carbon atoms may include a cyclopropylthio group, a cyclobutylthio group, a cyclopentylthio group, and a cyclohexylthio group.

The aralkyl group, the aralkoxy group, the aryloxy group, the aryl group, and the heteroaryl group may each be unsubstituted. In addition, 1 to 6 hydrogen atoms, particularly preferably 1 to 4 hydrogen atoms of each of the groups may each be substituted with a substituent selected from a hydroxyl group, an alkyl group, a haloalkyl group, a cycloalkyl group, an alkoxy group, an amino group, a heterocyclic group, a cyano group, a nitro group, and a halogen atom. Examples of the substituent include the same groups as the groups described in the section <$R^1$ and $R^2$>. The number of carbon atoms limited in each of the aralkyl group, the aralkoxy group, the aryloxy group, the aryl group, and the heteroaryl group does not include the number of carbon atoms of a substituent.

<Particularly Suitable $R^3$>

In consideration of, for example, the developed color tone and color development density of a photochromic monomer to be obtained, $R^3$ preferably represents a hydrogen atom, the alkyl group, the alkoxy group, the aryl group, or the arylthio group out of such groups as described above.

<$R^4$>

$R^4$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, an arylthio group having 6 to 10 carbon atoms that may have a substituent, a nitro group, a formyl group, a hydroxycarbonyl

group, an alkylcarbonyl group having 2 to 7 carbon atoms, an alkoxycarbonyl group having 2 to 7 carbon atoms, an aralkyl group having 7 to 11 carbon atoms that may have a substituent, an aralkoxy group having 7 to 11 carbon atoms that may have a substituent, an aryloxy group having 6 to 12 carbon atoms that may have a substituent, an aryl group having 6 to 12 carbon atoms that may have a substituent, a heteroaryl group having 3 to 12 carbon atoms that may have a substituent, a thiol group, an alkoxyalkylthio group having 2 to 9 carbon atoms, a haloalkylthio group having 1 to 6 carbon atoms, or a cycloalkylthio group having 3 to 8 carbon atoms.

Specific examples of the alkyl group having 1 to 6 carbon atoms, the haloalkyl group having 1 to 6 carbon atoms, the cycloalkyl group having 3 to 8 carbon atoms, the alkoxy group having 1 to 6 carbon atoms, the amino group, the heterocyclic group, the cyano group, the halogen atom, the alkylthio group having 1 to 6 carbon atoms, the arylthio group having 6 to 10 carbon atoms that may have a substituent, the alkylcarbonyl group having 2 to 7 carbon atoms, the alkoxycarbonyl group having 2 to 7 carbon atoms, the aralkyl group having 7 to 11 carbon atoms that may have a substituent, the aralkoxy group having 7 to 11 carbon atoms that may have a substituent, the aryloxy group having 6 to 12 carbon atoms that may have a substituent, the aryl group having 6 to 12 carbon atoms that may have a substituent, the heteroaryl group having 3 to 12 carbon atoms that may have a substituent, the alkoxyalkylthio group having 2 to 9 carbon atoms, the haloalkylthio group having 1 to 6 carbon atoms, or the cycloalkylthio group having 3 to 8 carbon atoms include specific groups listed in the section <R$^1$ and R$^2$> or the section <R$^3$>, and preferred groups thereof are also the same.

In addition, R$^3$ and R$^4$ may form a group represented by the following formula (3) together:

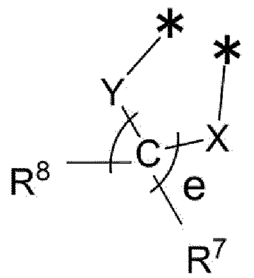

(3)

where * represents a carbon atom at a 6-position or a 7-position thereof.

In the formula, one, or each of both, of X and Y represents a sulfur atom, a methylene group, an oxygen atom, or a group represented by the following formula.

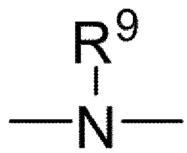

In the formula, R$^9$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an aryl group having 6 to 12 carbon atoms that may have a substituent, or a heteroaryl group having 3 to 12 carbon atoms that may have a substituent. Specific examples of those groups include specific groups listed in the section <R$^1$ and R$^2$> or the section <R$^3$>, and preferred groups thereof are also the same.

In addition, in the formula (3), R$^7$ and R$^8$ each independently represent a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a cyano group, a nitro group, a formyl group, a hydroxycarbonyl group, an alkylcarbonyl group having 2 to 7 carbon atoms, an alkoxycarbonyl group having 2 to 7 carbon atoms, a halogen atom, an aralkyl group having 7 to 11 carbon atoms that may have a substituent, an aralkoxy group having 7 to 11 carbon atoms that may have a substituent, an aryl group having 6 to 12 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent. Specific examples of those groups include specific groups listed in the section <R$^1$ and R$^2$> or the section <R$^3$>, and preferred groups thereof are also the same.

In addition, R$^7$ and R$^8$ may form an aliphatic ring together with a carbon atom to which R$^7$ and R$^8$ are bonded. Specific examples of the aliphatic ring include a cyclopentyl ring and a cyclohexyl ring.

In the formula, "e" represents an integer of from 1 to 3.

<Particularly Suitable $R^4$>

In consideration of, for example, the developed color tone and color development density of a photochromic monomer to be obtained, $R^4$ preferably represents a hydrogen atom, the alkyl group, the alkoxy group, the heterocyclic group, the aryl group, or the arylthio group out of such groups as described above.

<$R^5$>

$R^5$ represents a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a cyano group, a nitro group, a formyl group, a hydroxycarbonyl group, an alkylcarbonyl group having 2 to 7 carbon atoms, an alkoxycarbonyl group having 2 to 7 carbon atoms, a halogen atom, an aralkyl group having 7 to 11 carbon atoms that may have a substituent, an aralkoxy group having 7 to 11 carbon atoms that may have a substituent, an aryl group having 6 to 12 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent.

"c" represents an integer of from 0 to 2, and when "c" represents 2, $R^5$s may represent groups identical to or different from each other.

Specific examples of the alkyl group having 1 to 6 carbon atoms, the haloalkyl group having 1 to 6 carbon atoms, the cycloalkyl group having 3 to 8 carbon atoms, the alkoxy group having 1 to 6 carbon atoms, the amino group, the heterocyclic group, the alkylcarbonyl group having 2 to 7 carbon atoms, the alkoxycarbonyl group having 2 to 7 carbon atoms, the halogen atom, the aralkyl group having 7 to 11 carbon atoms that may have a substituent, the aralkoxy group having 7 to 11 carbon atoms that may have a substituent, the aryl group having 6 to 12 carbon atoms that may have a substituent, the thiol group, the alkylthio group having 1 to 6 carbon atoms, the alkoxyalkylthio group having 2 to 9 carbon atoms, the haloalkylthio group having 1 to 6 carbon atoms, the cycloalkylthio group having 3 to 8 carbon atoms, or the arylthio group having 6 to 10 carbon atoms that may have a substituent include specific groups listed in the section <$R^1$ and $R^2$> or the section <$R^3$> and the section <$R^4$>, and preferred groups thereof are also the same.

<Particularly Suitable $R^5$>

In consideration of, for example, the developed color tone and color development density of a photochromic monomer to be obtained, $R^5$ preferably represents a hydrogen atom (a case in which c=0) or the alkoxy group out of such groups as described above.

<$R^6$>

$R^6$ represents a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a halogen atom, an aralkyl group having 7 to 11 carbon atoms that may have a substituent, an aralkoxy group having 7 to 11 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent.

"d" represents an integer of from 0 to 4, and when "d" represents 2 or more, $R^6$S may represent groups identical to or different from each other.

Specific examples of the alkyl group having 1 to 6 carbon atoms, the haloalkyl group having 1 to 6 carbon atoms, the cycloalkyl group having 3 to 8 carbon atoms, the alkoxy group having 1 to 6 carbon atoms, the amino group, the heterocyclic group, the halogen atom, the aralkyl group having 7 to 11 carbon atoms that may have a substituent, the aralkoxy group having 7 to 11 carbon atoms that may have a substituent, the thiol group, the alkylthio group having 1 to 6 carbon atoms, the alkoxyalkylthio group having 2 to 9 carbon atoms, the haloalkylthio group having 1 to 6 carbon atoms, the cycloalkylthio group having 3 to 8 carbon atoms, or the arylthio group having 6 to 10 carbon atoms that may have a substituent include specific groups listed in the section <$R^1$ and $R^2$>, the section <$R^3$>, or the section <$R^4$>, and the section <$R^5$>, and preferred groups thereof are also the same.

<Particularly Suitable $R^6$>

In consideration of, for example, the developed color tone and color development density of a photochromic

monomer to be obtained, $R^6$ preferably represents a hydrogen atom (a case in which d=0) or the alkoxy group out of such groups as described above.

<Particularly Suitable Photochromic Monomer>

Specific examples of a particularly suitable photochromic monomer in the present invention include photochromic monomers represented by the following formulae.

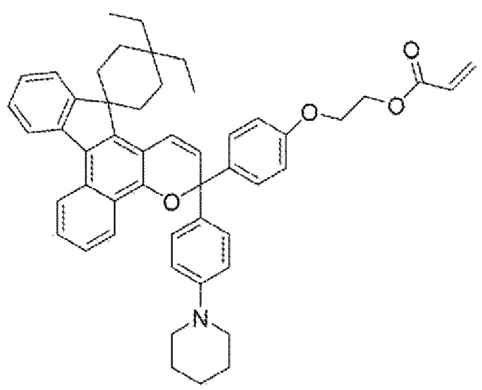

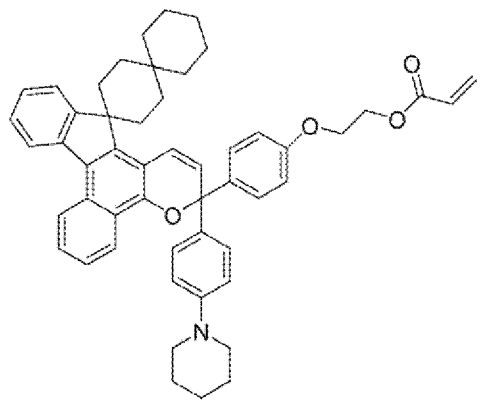

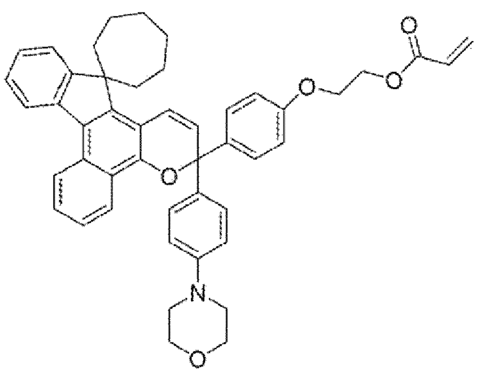

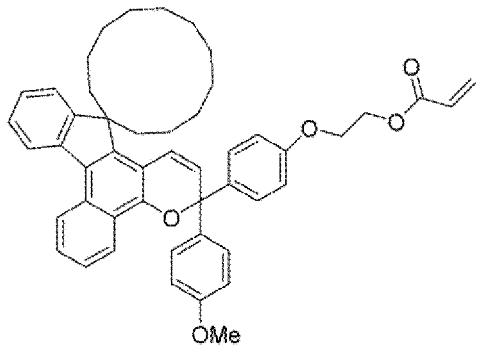

<Identification of Suitable Photochromic Monomer>

The photochromic monomer generally exists as a colorless, pale yellow, or pale green solid or viscous liquid at normal temperature and normal pressure, and can be identified by the following methods (a) to (c).

(a) When the proton nuclear magnetic resonance spectrum ($^1$H-NMR) of the photochromic monomer is measured, a peak based on an aromatic proton and the proton of an alkene appears at a $\delta$ of from about 5.0 ppm to about 9.0 ppm, and a peak based on the protons of an alkyl group and an alkylene group appears at a $\delta$ of from about 1.0 ppm to about 4.0 ppm. In addition, the number of protons of each bonding group can be understood by relatively comparing the spectral intensities of the respective peaks.

(b) The composition of a corresponding product can be determined by elemental analysis.

(c) When the $^{13}$C nuclear magnetic resonance spectrum ($^{13}$C-NMR) of the photochromic monomer is measured, a peak based on the carbon atoms of an aromatic hydrocarbon group appears at a $\delta$ of from about 110 ppm to about 160 ppm, a peak based on the carbon atoms of an alkene and an alkyne appears at a $\delta$ of from about 80 ppm to about 140 ppm, and a peak based on the carbon atoms of an alkyl group and an alkylene group appears at a $\delta$ of from about 20 ppm to about 80 ppm.

<Production of Suitable Photochromic Monomer>

A suitable photochromic monomer to be used in the present invention may be produced by any synthesis

method. An example of a method of producing the suitable photochromic monomer is described. In the following description, the symbols in the respective formulae each represent the same meaning as that described for the above-mentioned formulae unless otherwise stated.

The photochromic monomer can be suitably produced by a method including causing a naphthol compound represented by the following formula (10):

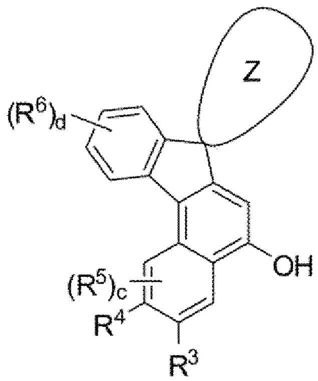

(10)

and a propargyl alcohol compound represented by the following formula (11) to react with each other in the presence of an acid catalyst.

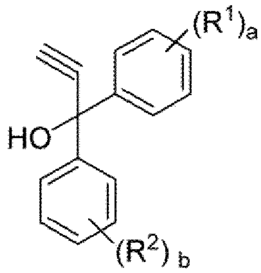

(11)

A reaction ratio between the naphthol compound and the propargyl alcohol compound is preferably selected from the range of from 1:10 to 10:1 (molar ratio). In addition, for example, sulfuric acid, benzenesulfonic acid, p-toluenesulfonic acid, or acidic alumina is used as the acid catalyst. The acid catalyst is preferably used in an amount in the range of from 0.1 part by weight to 10 parts by weight per 100 parts by weight of the sum of the naphthol compound and the propargyl alcohol compound. A reaction temperature is preferably from 0°C to 200°C. An aprotic organic solvent, such as N-methylpyrrolidone, dimethylformamide, tetrahydrofuran, benzene, or toluene, is preferably used as a solvent. A method of purifying the product obtained by such reaction is not particularly limited. For example, the product may be purified by purifying the product with a silica gel column and recrystallizing the purified product.

Of the naphthol compounds each represented by the formula (10), a compound having a structure enabling the production of the preferred photochromic monomer represented by the formula (1) is a preferred compound. For example, compounds represented by the following formulae may be listed as particularly preferred compounds.

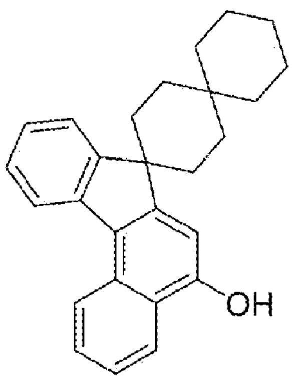

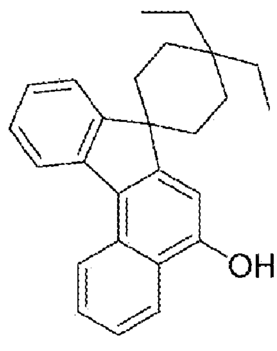

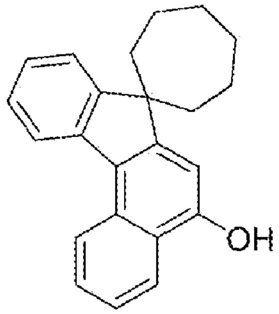

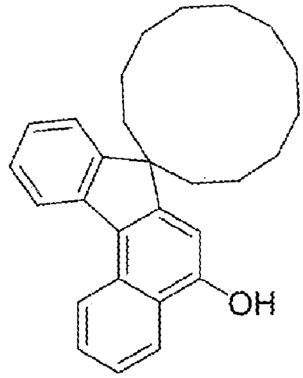

The naphthol compound represented by the formula (10) may be synthesized, for example, as described below. The naphthol compound may be synthesized on the basis of a reaction method described in a thesis, such as WO 2001/60881 A2 or WO 2005/028465 A1. Specifically, the naphthol compound may be produced by the following method.

First, a benzophenone compound represented by the following formula (12):

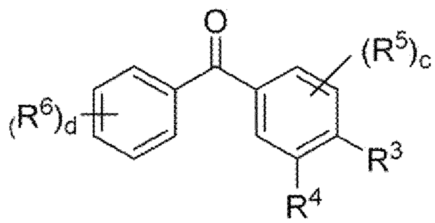

(12)

is subjected to a Stobbe reaction and a cyclization reaction to provide a compound represented by the following formula (13).

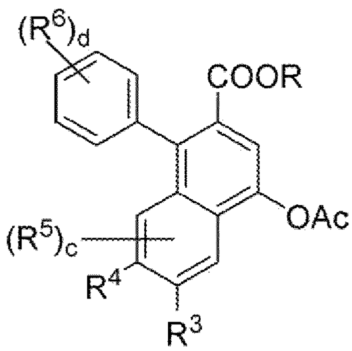

(13)

In the compound represented by the formula (13), R represents a group derived from a diester compound used in the Stobbe reaction, and Ac represents an acetyl group. Next, the compound (13) is hydrolyzed with an alkali or an acid to provide a carboxylic acid represented by the following formula (14).

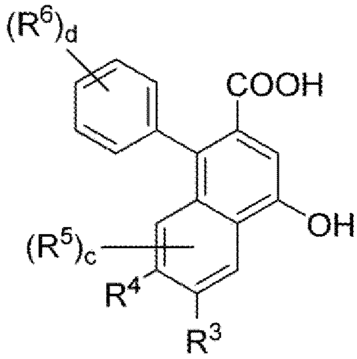

(14)

The carboxylic acid is subjected to benzylation with a base, such as potassium carbonate, and benzyl chloride, and is then hydrolyzed with an alkali or an acid to provide a benzyl-protected carboxylic acid represented by the following formula (15) :

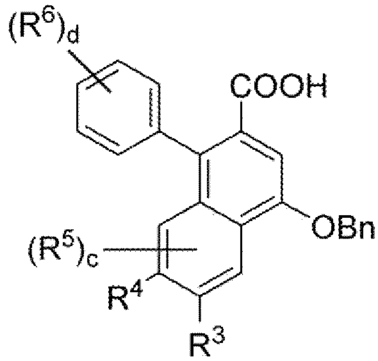

(15)

(in the formula (15), Bn represents a benzyl group). The benzyl-protected carboxylic acid is converted into an amine by a method such as Curtius rearrangement, Hofmann rearrangement, or Lossen rearrangement, and a diazonium salt is prepared from the amine by a method known per se. The diazonium salt is converted into a bromide by a Sandmeyer reaction or the like, and the resultant bromide is caused to react with magnesium, lithium, or the like to prepare an organometallic compound. The organometallic compound is caused to react with a ketone represented by the following formula (16):

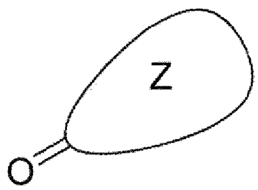

(16)

in an organic solvent at from -80°C to 70°C for from 10 minutes to 4 hours, and then the resultant is subjected to a debenzylation reaction with hydrogen and palladium carbon or the like to provide an alcohol represented by the following formula (17) .

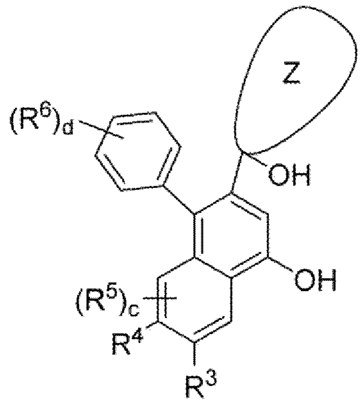

(17)

The alcohol is subjected to a Friedel-Crafts reaction under a neutral to acidic condition at from 10°C to 120°C for from 10 minutes to 2 hours. Thus, the target naphthol compound represented by the formula (10) can be synthesized. In such reaction, a reaction ratio between the organometallic compound and the ketone represented by the formula (16) is preferably selected from the range of from 1:10 to 10:1 (molar ratio). A reaction temperature is preferably from -80°C to 70°C. An aprotic organic solvent, such as diethyl ether, tetrahydrofuran, benzene, or toluene, is preferably used as the solvent. In addition, the Friedel-Crafts reaction of the alcohol represented by the formula (17) under the neutral to acidic condition is preferably performed by using an acid catalyst, such as acetic acid, hydrochloric acid, sulfuric acid, benzenesulfonic acid, p-toluenesulfonic acid, or acidic alumina. Such acid catalyst is suitably used in an amount in the range of from 0.1 part by weight to 10 parts by weight per 100 parts by weight of the alcohol represented by the formula (17). An aprotic organic solvent, such as tetrahydrofuran, benzene, or toluene, is used at the time of the reaction.

Meanwhile, the propargyl alcohol compound represented by the formula (11) may be easily synthesized by, for example, causing a ketone compound corresponding to the formula (11) and a metal acetylene compound, such as lithium acetylide, to react with each other. When the polymerizable group is introduced into the propargyl alcohol compound represented by the formula (11) by a known method, the photochromic monomer can be produced by performing the reaction between the propargyl alcohol compound and the naphthol compound represented by the formula (10).

Although the photochromic monomer may be produced by such method as described above, to further simplify the reactions and to suppress a by-product, the radical-polymerizable group is preferably introduced as described below. Specifically, the position of the propargyl alcohol compound into which the radical-polymerizable group is to be introduced is substituted with a reactive substituent, such as a hydroxyl group, a primary or secondary amino group, a thiol group, or a hydrosilyl group, in advance. Next, the propargyl alcohol compound having the reactive substituent and the naphthol compound represented by the formula (10) are caused to react with each other in accordance with the foregoing method to produce a precursor of the photochromic monomer. Then, the radical-polymerizable group is introduced into the reactive substituent of the resultant precursor to produce the photochromic monomer.

A known method may be adopted as a method of introducing the radical-polymerizable group into the reactive substituent.

For example, when a (meth)acrylic group is introduced as the radical-polymerizable group, a precursor having a hydroxyl group as the reactive substituent and (meth) acryloyl chloride only need to be caused to react with each other in the presence of a basic catalyst. In addition to the foregoing, the radical-polymerizable group may be introduced by causing a precursor having an amino group or a hydroxyl group and 2-isocyanatoethyl (meth)acrylate to react with each other. In addition, the radical-polymerizable group may be introduced by hydrosilylating a precursor having a hydrosilyl group as the reactive substituent and allyl methacrylate through use of chloroplatinic acid as a catalyst.

In addition, when a vinyl group is introduced as the radical-polymerizable group, the radical-polymerizable group may be introduced by causing a precursor having a hydroxyl group as the reactive substituent and vinyl chloride or allyl bromide to react with each other.

When the radical-polymerizable group is a styryl group, the radical-polymerizable group may be introduced by hydrosilylating a precursor having a hydrosilyl group as the reactive substituent and divinylbenzene in the presence of a chloroplatinic acid catalyst.

A more specific example of the production method is described below. For example, a method of synthesizing a precursor having a hydroxyl group as the reactive substituent and a conversion scheme when an acrylic group is introduced as the radical-polymerizable group into the precursor are described below.

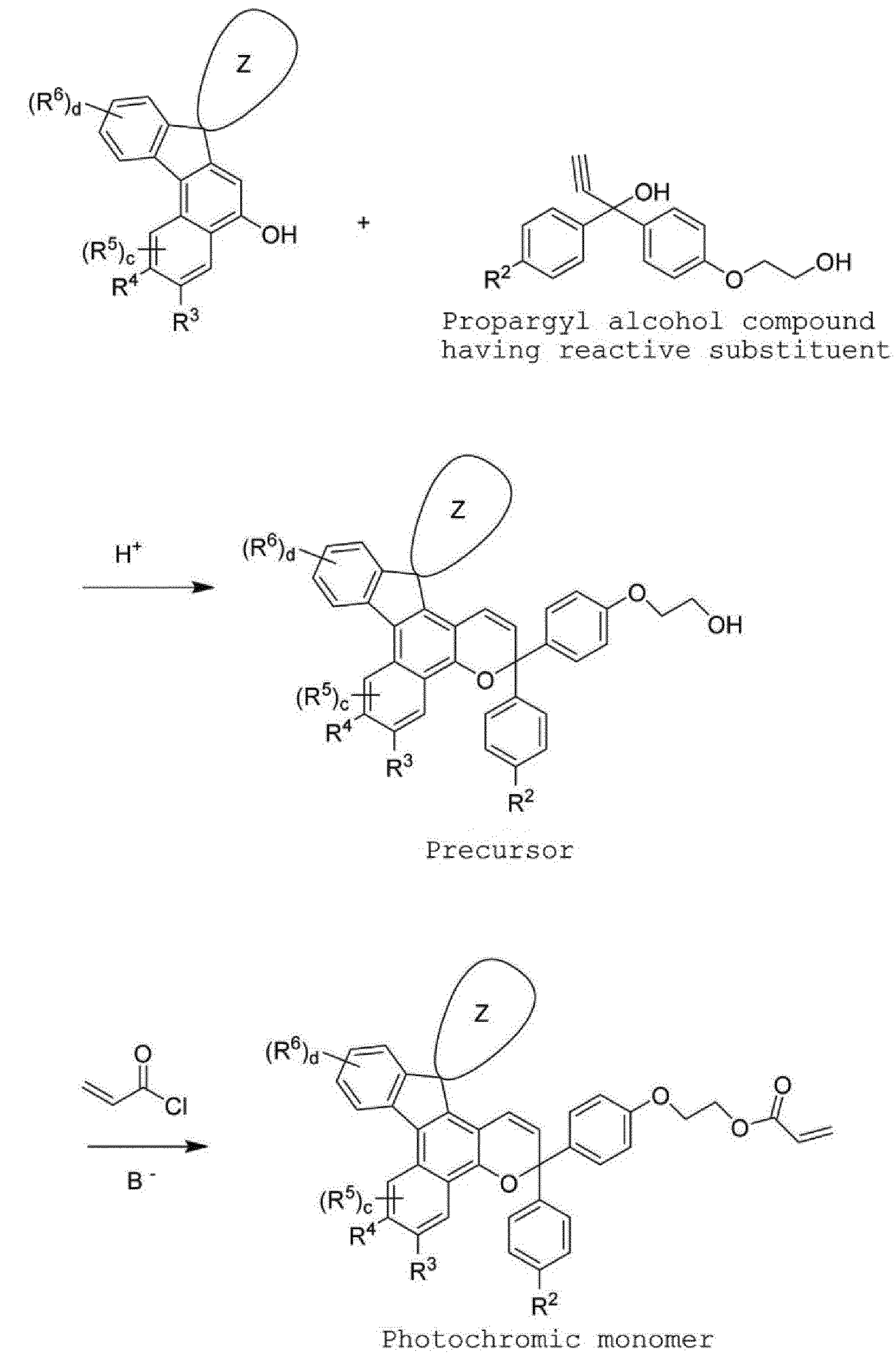


The precursor having a hydroxyl group may be obtained by causing the naphthol compound represented by the formula (10) and a propargyl alcohol compound having a hydroxyl group as the reactive substituent to react with each other under an acidic condition. The photochromic monomer is obtained by causing the precursor to react with acryloyl chloride in the presence of a basic catalyst, such as a tertiary amine.

A plurality of kinds of photochromic monomers may be used as the photochromic monomers for obtaining a desired color tone. When the plurality of kinds of photochromic monomers are used, as a matter of course, the following content ratio is based on the total amount of the photochromic monomers.

The content ratio of the photochromic monomer in the monomer components may be typically from 0.001 wt%

to 5 wt%, preferably from 0.01 wt% to 3 wt%, though the content ratio may vary depending on the absorption wavelength of the monomer and the magnitude of absorption thereof. When the ophthalmic medical device is used in a hydrogel soft contact lens application, the content ratio is preferably from 0.3 wt% to 3 wt%, more preferably from 0.5 wt% to 2 wt%, still more preferably from 0.6 wt% to 1.5 wt%. In addition, when the ophthalmic medical device is used in the application of a lens substantially free of water (hereinafter sometimes referred to as "non-hydrogel lens"), such as an oxygen-permeable hard contact lens, a non-hydrogel soft contact lens, or an intraocular lens, the content ratio is preferably from 0.01 wt% to 3 wt%, more preferably from 0.1 wt% to 2 wt%. When the content ratio falls within the ranges, an ophthalmic medical device having a desired photochromic property can be suitably obtained.

B-2. Lactam Ring-containing Monomer

The lactam ring-containing monomer can improve the compatibility of the photochromic monomer in the monomer components, and hence can provide a photochromic polymer excellent in transparency. Examples of the lactam ring-containing monomer include an N-vinyl lactam and a methylene lactam. Of those, an N-vinyl lactam may be preferably used. The N-vinyl lactam is preferred because of the following reasons: the N-vinyl lactam can impart hydrophilicity to the photochromic polymer; the N-vinyl lactam is excellent in biosafety; and the N-vinyl lactam is available at low cost. The lactam ring-containing monomers may be used alone or in combination thereof.

Examples of the N-vinyl lactam include N-vinyl-2-pyrrolidone, N-vinyl-3-methyl-2-pyrrolidone, N-vinyl-4-methyl-2-pyrrolidone, N-vinyl-5-methyl-2-pyrrolidone, N-vinyl-6-methyl-2-pyrrolidone, N-vinyl-3-ethyl-2-pyrrolidone, N-vinyl-4,5-dimethyl-2-pyrrolidone, N-vinyl-5,5-dimethyl-2-pyrrolidone, N-vinyl-3,3,5-trimethyl-2-pyrrolidone, N-vinyl-2-piperidone, N-vinyl-3-methyl-2-piperidone, N-vinyl-4-methyl-2-piperidone, N-vinyl-5-methyl-2-piperidone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-2-caprolactam, N-vinyl-3-methyl-2-caprolactam, N-vinyl-4-methyl-2-caprolactam, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam, and N-vinyl-3,5,7-trimethyl-2-caprolactam. Of those, N-vinyl-2-pyrrolidone and N-vinyl-2-caprolactam are preferred, and N-vinyl-2-pyrrolidone is more preferred.

Examples of the methylene lactam include methylene pyrrolidones, such as 1-methyl-3-methylene-2-pyrrolidone, 1-ethyl-3-methylene-2-pyrrolidone, 1-methyl-5-methylene-2-pyrrolidone, 1-ethyl-5-methylene-2-pyrrolidone, 5-methyl-3-methylene-2-pyrrolidone, 5-ethyl-3-methylene-2-pyrrolidone, 1-n-propyl-3-methylene-2-pyrrolidone, 1-n-propyl-5-methylene-2-pyrrolidone, 1-i-propyl-3-methylene-2-pyrrolidone, 1-i-propyl-5-methylene-2-pyrrolidone, 1-n-butyl-3-methylene-2-pyrrolidone, and 1-t-butyl-3-methylene-2-pyrrolidone.

The content ratio of the lactam ring-containing monomer in the monomer components is typically from 10 wt% to 50 wt%, preferably from 11 wt% to 48 wt%, more preferably from 12 wt% to 45 wt%. When the content ratio falls within the ranges, there can be obtained a photochromic polymer excellent in transparency even when no organic solvent is used or an organic solvent is used in a small amount.

B-3. Silicone-containing Monomer

The silicone-containing monomer can contribute to improvements in, for example, oxygen permeability, mechanical strength, and shape stability of the photochromic polymer. The silicone-containing monomer is not particularly limited as long as the monomer contains a siloxane bond (Si-O-Si), and examples thereof include a silicone-containing alkyl (meth)acrylate, a silicone-containing styrene derivative, and a silicon-containing fumaric acid diester. In addition, the silicone-containing monomer may be a silicone-containing macromonomer having a polysiloxane structure.

Specific examples of the silicone-containing alkyl (meth)acrylate include trimethylsiloxydimethylsilylmethyl (meth)acrylate, trimethylsiloxydimethylsilylpropyl (meth)acrylate, methylbis(trimethylsiloxy)silylpropyl (meth)acrylate, tris(trimethylsiloxy)silylpropyl (meth)acrylate, mono[methylbis(trimethylsiloxy)siloxy]bis(trimethylsiloxy)sily lpropyl (meth)acrylate, tris[methylbis(trimethylsiloxy)siloxy]silylpropyl (meth)acrylate, tris(trimethylsiloxy)silyl(propylglycerol) (meth)acrylate, and polydimethyl siloxane di(meth)acrylate.

Specific examples of the silicone-containing styrene derivative include tris(trimethylsiloxy)silylstyrene, bis(trimethylsiloxy)methylsilylstyrene, (trimethylsiloxy)dimethylsilylstyrene, and tris(trimethylsiloxy)siloxydimethylsilylstyrene.

Specific examples of the silicone-containing fumaric acid diester include bis(3-(trimethylsilyl)propyl fumarate, bis(3-(pentamethyldisiloxanyl)propyl) fumarate, bis(3-(1,3,3,3-tetramethyl-1-(trimethylsilyl)oxy)disiloxanyl)propyl) fumarate, and bis(tris(trimethylsiloxy)silylpropyl) fumarate.

A silicone-containing macromonomer having a polysiloxane structure in which the number of repetitions of (Si-O) is, for example, 4 or more, preferably from 4 to 200, more preferably from 10 to 200 may be preferably used as the silicone-containing macromonomer. The use of such silicone-containing macromonomer can achieve a high oxygen permeability. Details about the silicone-containing macromonomer are described in, for example, JP 2011-219513 A and JP 2015-503631 A, which are incorporated herein by reference.

The content ratio of the silicone-containing monomer in the monomer components is appropriately set in accordance with, for example, physical properties that the ophthalmic medical device is desired to have and its applications. The content may be, for example, from 10 wt% to 89.9 wt%. Specifically, when the ophthalmic medical device is used in a hydrogel soft contact lens application, the content ratio is preferably from 25 wt% to 60 wt%, more preferably from 28 wt% to 60 wt%. Meanwhile, when the ophthalmic medical device is used in a non-hydrogel lens application, the content ratio is preferably from 12 wt% to 89.9 wt%, more preferably from 15 wt% to 80 wt%. When the content ratio of the silicone-containing monomer falls within the ranges, a photochromic polymer (consequently, an ophthalmic medical device) having desired oxygen permeability and desired transparency can be suitably obtained.

B-4. Hydrophilic Monomer

The hydrophilic monomer can improve the hydrophilicity of the photochromic polymer, and can improve the water wettability and lubricity of the surface thereof. For example, a monomer having a solubility in water at 20°C of 20 g/100 mL or more (provided that the monomers described in the section B-1 to the section B-3 and the section B-5 are excluded) may be preferably used as the hydrophilic monomer. Preferred specific examples of the hydrophilic monomer may include: amide group-containing monomers, such as a (meth)acrylamide-based compound and N-vinylamide; and hydroxyl group-containing monomers, such as hydroxyalkyl (meth)acrylate or dihydroxyalkyl (meth)acrylate each having 1 to 5 carbon atoms. The hydrophilic monomers may be used alone or in combination thereof.

The (meth)acrylamide-based compound is preferably used as the hydrophilic monomer. When the N-vinyl lactam is used as the lactam ring-containing monomer, the photochromic polymer to be obtained may yellow or a polymerization failure may occur. Although a reason for the foregoing is unclear, the reason is assumed to be as described below. That is, the N-vinyl lactam has a relatively slow polymerization rate and has high polarity, and hence interacts with the photochromic monomer activated by exposure at the time of its polymerization. It is assumed that as a result of the foregoing, the photochromic monomer may decompose to cause a problem, such as the yellowing of an entire system or the suppression of the polymerization. In contrast, combined use of the (meth)acrylamide-based compound with the N-vinyl lactam can suppress the yellowing of the photochromic polymer and the polymerization failure. The combined use of the N-vinyl lactam and the (meth)acrylamide-based compound increases the viscosity of the polymerization system (reactive mixture) to suppress the diffusion of the N-vinyl lactam remaining in the later stage of the polymerization. Probably as a result of the foregoing, the interaction with the photochromic monomer (consequently, the deterioration of the photochromic monomer) is also suppressed.

Examples of the (meth)acrylamide-based compound include: (meth)acrylamide; N-alkyl(meth)acrylamides, such as N-methyl(meth)acrylamide and N-ethyl(meth)acrylamide; N,N-dialkyl(meth)acrylamides, such as N,N-dimethyl(meth)acrylamide, N,N-diethyl(meth)acrylamide, and N,N-dipropyl(meth)acrylamide; N,N-dialkylaminoalkyl(meth)acrylamides, such as N,N-dimethylaminopropyl(meth)acrylamide and N,N-diethylaminopropyl(meth)acrylamide; and acryloylmorpholine.

Examples of the N-vinylamide include N-vinylformamide, N-vinyl-N-methylformamide, N-vinyl-N-ethylformamide, N-vinylacetamide, N-vinyl-N-methylacetamide, N-vinyl-N-ethylacetamide, and N-vinylphthalimide.

Examples of the hydroxyalkyl (meth)acrylate having 1 to 5 carbon atoms include hydroxyethyl (meth)acrylate, hydroxypropyl (meth)acrylate, hydroxybutyl (meth)acrylate, and hydroxypentyl (meth)acrylate.

Examples of the dihydroxyalkyl (meth)acrylate having 1 to 5 carbon atoms include dihydroxypropyl (meth)acrylate, dihydroxybutyl (meth)acrylate, and dihydroxypentyl (meth)acrylate.

Examples of the hydrophilic monomer except the above-mentioned monomers may include an alkoxypolyalkylene glycol mono(meth)acrylate, (meth)acrylic acid, 1-methyl-3-methylene-2-pyrrolidinone, maleic anhydride, maleic acid, a maleic acid derivative, fumaric acid, a fumaric acid derivative, and aminostyrene.

The content ratio of the hydrophilic monomer in the monomer components is appropriately set in accordance with, for example, the content ratio of the lactam ring-containing monomer, and physical properties that the ophthalmic medical device is desired to have and its applications. For example, the hydrophilic monomer may be blended so that the total content ratio of the monomer and the lactam ring-containing monomer in the monomer components may be more than 10 wt% and 65 wt% or less. Specifically, when the ophthalmic medical device is used in a hydrogel soft contact lens application, the hydrophilic monomer is blended so that the total content ratio of the monomer and the lactam ring-containing monomer in the monomer components may be preferably from 25 wt% to 65 wt%, more preferably from 30 wt% to 60 wt%, still more preferably from 35 wt% to 60 wt%. In addition, when the ophthalmic medical device is used in a non-hydrogel lens application, the hydrophilic monomer is blended so that the total content ratio of the monomer and the lactam ring-containing monomer in the monomer components may be, for example, more than 10 wt% and less than 25 wt%, preferably more than 10 wt% and 20 wt% or less, more preferably more than 10 wt% and 15 wt% or less. When the content ratio falls within the ranges, the compatibility of the hydrophilic monomer with the photochromic monomer is secured, and hence a photochromic polymer having high transparency can be obtained.

When the hydrophilic monomer contains the (meth)acrylamide-based compound, the blending amount of the

(meth)acrylamide-based compound is preferably 5 wt% or more, more preferably from 5 wt% to 50 wt%, still more preferably from 10 wt% to 40 wt% with respect to the blending amount of the lactam ring-containing monomer. In addition, when the ophthalmic medical device is used in a hydrogel soft contact lens application, the total content ratio of the lactam ring-containing monomer and the (meth)acrylamide-based compound in the monomer components is preferably from 25 wt% to 65 wt%, more preferably from 30 wt% to 60 wt%, still more preferably from 35 wt% to 55 wt%. In addition, when the ophthalmic medical device is used in a non-hydrogel lens application, the total content ratio of the lactam ring-containing monomer and the (meth)acrylamide-based compound in the monomer components is, for example, more than 10 wt% and less than 25 wt%, preferably from 11 wt% to 20 wt%, more preferably from 12 wt% to 15 wt%.

When the hydrophilic monomer contains the hydroxyl group-containing monomer, the content ratio of the hydroxyl group-containing monomer in the total content of the hydrophilic monomer and the lactam ring-containing monomer is preferably 35 wt% or less, more preferably 30 wt% or less, still more preferably 20 wt% or less, yet still more preferably 10 wt% or less. When the content ratio falls within the ranges, the compatibility of the hydrophilic monomer with the photochromic monomer is secured, and hence a photochromic polymer having high transparency can be suitably obtained. In one embodiment, the content ratio of the hydroxyl group-containing monomer in the monomer components is preferably 20 wt% or less, more preferably 15 wt% or less, still more preferably 10 wt% or less.

B-5. Cross-linkable Monomer

The cross-linkable monomer can improve the mechanical strength of the photochromic polymer. A monomer having two or more polymerizable functional groups is used as the cross-linkable monomer.

Specific examples of the cross-linkable monomer include butanediol di(meth)acrylate, ethylene glycol di(meth)acrylate, diethylene glycol di(meth)acrylate, triethylene glycol di(meth)acrylate, propylene glycol di(meth)acrylate, dipropylene glycol di(meth)acrylate, diallyl fumarate, allyl (meth)acrylate, vinyl (meth)acrylate, trimethylolpropane tri(meth)acrylate, methacryloyloxyethyl (meth)acrylate, divinylbenzene, diallyl phthalate, diallyl adipate, triallyl diisocyanate, $\alpha$-methylene-N-vinylpyrrolidone, 4-vinylbenzyl (meth)acrylate, 3-vinylbenzyl (meth)acrylate, 2,2-bis((meth)acryloyloxyphenyl)hexafluoropropane, 2,2-bis((meth)acryloyloxyphenyl)propane, 1,4-bis(2-(meth)acryloyloxyhexafluoroisopropyl)benzene, 1,3-bis(2-(meth)acryloyloxyhexafluoroisopropyl)benzene, 1,2-bis(2-(meth)acryloyloxyhexafluoroisopropyl)benzene, 1,4-bis(2-(meth)acryloyloxyisopropyl)benzene, 1,3-bis(2-(meth)acryloyloxyisopropyl)benzene, and 1,2-bis(2-(meth)acryloyloxyisopropyl)benzene. The cross-linkable monomers may be used alone or in combination thereof.

The content ratio of the cross-linkable monomer in the monomer components is appropriately set in accordance with, for example, physical properties that the ophthalmic medical device is desired to have and its applications. The content may be, for example, from 0.01 wt% to 18 wt%. Specifically, when the ophthalmic medical device is used in a hydrogel soft contact lens application, the content ratio may be set to, for example, from 0.1 wt% to 3 wt%, preferably from 0.2 wt% to 2 wt%. When the ophthalmic medical device is used in an oxygen-permeable hard contact lens application, the content ratio is preferably from 3 wt% to 18 wt%, more preferably from 5 wt% to 15 wt%. When the device is used in a non-hydrogel soft contact lens application, the content ratio is preferably from 0.01 wt% to 15 wt%, more preferably from 0.1 wt% to 10 wt%. When the device is used in an intraocular lens application, the content ratio is preferably from 1 wt% to 5 wt%, more preferably from 2 wt% to 4 wt%. When the content ratio of the cross-linkable monomer falls within the ranges, a photochromic polymer having satisfactory mechanical strength can be obtained.

B-6. Other Copolymerizable Monomer

The monomer components may further include a copolymerizable monomer except those described in the section B-1 to the section B-5. Any appropriate monomer may be selected as the copolymerizable monomer in accordance with purposes. Specific examples thereof include a hydrophobic monomer, a polymerizable color additive, and a polymerizable UV absorber. Herein, a monomer having a water solubility (20°C) of less than 20 g/100 mL (provided that the monomers described in the section B-1 to the section B-5 are excluded) may be used as the hydrophobic monomer, and a prepolymer prepared by using various monomers and cross-linkable monomers described in the section B-1 to the section B-6 may also be used as the hydrophobic monomer as long as the prepolymer has a water solubility (20°C) of less than 20 g/100 mL.

Examples of the hydrophobic monomer include an alkyl (meth)acrylate, an alkoxyalkyl (meth)acrylate, and an aromatic ring-containing (meth)acrylate. The hydrophobic monomers may be used alone or in combination thereof.

A linear or branched alkyl (meth)acrylate whose alkyl group has 1 to 20 carbon atoms, or a fluorine-substituted monomer thereof is preferred as the alkyl (meth)acrylate, and a linear or branched alkyl (meth)acrylate whose alkyl group has 1 to 5 carbon atoms, or a fluorine-substituted monomer thereof is more preferred. Specific examples thereof include methyl (meth)acrylate, ethyl (meth)acrylate, n-propyl (meth)acrylate, isopropyl (meth)acrylate, butyl (meth)acrylate, ethylhexyl (meth)acrylate, and lauryl (meth)acrylate.

An alkoxyalkyl (meth)acrylate in which the number of carbon atoms of an alkoxy group is from 1 to 4 and the number of carbon atoms of an alkylene group bonded to the alkoxy group is from 1 to 8 is preferred as the alkoxyalkyl (meth)acrylate, and an alkoxyalkyl (meth) acrylate in which the number of carbon atoms of an alkoxy group is 1 or 2 and the number of carbon atoms of an alkylene group bonded to the alkoxy group is from 1 to 4 is more preferred. Specific examples thereof include methoxymethyl (meth)acrylate, 2-methoxyethyl (meth)acrylate, ethoxymethyl (meth)acrylate, 2-ethoxyethyl (meth)acrylate, 3-methoxypropyl (meth)acrylate, 3-ethoxypropyl (meth)acrylate, 4-methoxybutyl (meth)acrylate, and 4-ethoxybutyl (meth)acrylate.

Examples of the aromatic ring-containing (meth)acrylate include phenoxyethyl (meth)acrylate, phenyl (meth)acrylate, and phenylethyl (meth)acrylate.

An example of the prepolymer prepared by using the monomers and the cross-linkable monomers is a prepolymer obtained by: causing such a monomer and a cross-linkable monomer as disclosed herein to coexist; and polymerizing the monomers with a polymerization initiator.

The content ratio of the hydrophobic monomer in the monomer components is appropriately set in accordance with, for example, physical properties that the ophthalmic medical device is desired to have and its applications. When the ophthalmic medical device is used in a hydrogel soft contact lens application, the content ratio is preferably from 5 wt% to 35 wt%, more preferably from 10 wt% to 30 wt%. Meanwhile, when the ophthalmic medical device is used in a non-hydrogel lens application, the content ratio is preferably from 5 wt% to 40 wt%, more preferably from 5 wt% to 35 wt%. When the content ratio falls within the ranges, an ophthalmic medical device having desired mechanical strength can be suitably obtained.

Examples of the polymerizable color additive include: phthalocyanine-based polymerizable color additives, such as a phthalocyanine-containing polymethacrylic acid ester; azo-based polymerizable color additives, such as 1-phenylazo-4-(meth)acryloyloxynaphthalene; anthraquinone-based polymerizable color additives, such as 1,5-bis((meth)acryloylamino)-9,10-anthraquinone, 1,4-bis(4-(2-methacryloxyethyl)phenylamino)anthraquinone (C.I. Reactive Blue 246), and 1,4-bis[(2-hydroxyethyl)amino]-9,10-anthracenedione bis(2-methyl-2-propenoic) ester (C.I. Reactive Blue 247); and nitro-based polymerizable color additives, such as o-nitroanilinomethyl (meth)acrylate. Of those, a phthalocyanine-based polymerizable color additive is preferred. Examples of the phthalocyanine-based polymerizable color additive include (meth)acryloylated tetraaminocopper phthalocyanine and (meth)acryloylated (dodecanoylated tetraaminocopper phthalocyanine). Further, a benzophenone-based polymerizable UV-absorbing color additive, such as 2,4-dihydroxy-3(p-stylenoazo)benzophenone, or a benzoic acid-based polymerizable UV-absorbing color additive, such as phenyl 2-hydroxy-4-(p-stylenoazo)benzoate, may also be used. Those polymerizable color additives may be used alone or as a mixture thereof.

Examples of the polymerizable UV absorber include: a compound having a benzotriazole structure and a polymerizable group (benzotriazole-based compound); a compound having a benzophenone structure and a polymerizable group (benzophenone-based compound); and a salicylic acid derivative compound. The monomer components including a UV-absorbing compound are preferred as materials for an ophthalmic medical device because the compound can block UV light. Of those components, a benzotriazole-based compound is preferred from the viewpoints of the characteristics of the compound, such as a UV-absorbing ability. Examples of the benzotriazole-based compound include 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, 2-(2'-hydroxy-5'-(meth)acryloyloxypropylphenyl)-2H-benzotriazole, 2-(2'-hydroxy-5'-(meth)acryloyloxypropyl-3'-t-butylphenyl)-5-chloro-2H-benzotriazole, and 2-(2'-hydroxy-5'-(2"-methacryloyloxyethoxy)-3'-t-butylphenyl)-5-methyl-2H-benzotriazole. Of those, 2-(2'-hydroxy-5'-(meth)acryloyloxyethylphenyl)-2H-benzotriazole is preferred. The polymerizable group of the benzotriazole-based compound is not particularly limited, but a methacryloyl group is preferred. Examples of the benzophenone-based compound include 2-hydroxy-4-(meth)acryloyloxybenzophenone, 2-hydroxy-4-(meth)acryloyloxy-5-t-butylbenzophenone, 2-hydroxy-4-(meth)acryloyloxy-2',4'-dichlorobenzophenone, and 2-hydroxy-4-(2'-hydroxy-3'-(meth)acryloyloxypropoxy)benzophenone. An example of the salicylic acid derivative compound is phenyl 2-hydroxy-4-methacryloyloxymethylbenzoate. Another example thereof is 2-cyano-3-phenyl-3-(3'-(meth)acryloyloxyphenyl)propenoic acid methyl ester. Those compounds may be used alone or as a mixture thereof.

The blending ratio of the polymerizable color additive in the monomer components may be set to, for example, from 0.001 wt% to 0.1 wt%, preferably from 0.002 wt% to 0.05 wt%. When the content is 0.001 wt% or less, there is a risk in that the coloring of the ophthalmic medical device exhibits no effect, and hence the visibility thereof is low, and when the content is 0.1 wt% or more, there is a risk in that the coloring is so dark that the field of view of a person at the time of his or her wearing of the device is inhibited.

The blending ratio of the polymerizable UV absorber in the monomer components is, for example, 3 wt% or less, preferably from 0.01 wt% to 2 wt%. When the content is 3 wt% or less, the deterioration of the mechanical characteristic (loss of the softness) of the photochromic polymer can be further suppressed, and when the content is 0.01 wt% or more, a UV-absorbing effect can be sufficiently obtained.

B-7. Polymerization Method

The photochromic polymer is obtained by polymerizing a reactive mixture containing the monomer components. The polymerization may be performed by, for example, mixing the monomer components and an initiator to prepare the reactive mixture, and subjecting the reactive mixture to heating and/or irradiation with light (UV light and/or visible light). Electron beam irradiation may be performed instead of the light irradiation.

In the preparation of the reactive mixture, an organic solvent and/or an additive may be added as required. When the organic solvent is added to the reactive mixture, the content ratio of the organic solvent in the reactive mixture is preferably 10 parts by weight or less, more preferably 5 parts by weight or less, still more preferably 3 parts by weight or less, yet still more preferably 1 part by weight or less, particularly preferably 0.5 part by weight or less with respect to 100 parts by weight of the monomer components. The following fact can be regarded as one effect of the present invention: the monomer components can be made compatible with each other without use of the organic solvent or by using a small amount thereof as described above, and as a result, a photochromic polymer excellent in transparency is obtained. In addition, when the polymerization reaction is performed without use of the organic solvent or by using a small amount thereof, a step of removing the organic solvent from the resultant photochromic polymer can be made unnecessary or simplified, and chain transfer at the time of the polymerization is suppressed. As a result, a photochromic polymer having a large polymerization degree and high strength can be obtained.

An alcohol having 1 to 3 carbon atoms, acetone, methyl ethyl ketone, dimethylformamide, dimethyl sulfoxide, acetonitrile, N-methyl-2-pyrrolidone, dimethoxyethane, or the like may be used as the organic solvent.

The kind of the initiator is appropriately selected in accordance with, for example, a polymerization method. For example, examples of the photopolymerization initiator to be used in the polymerization through light irradiation (photopolymerization) include: phosphine oxide-based photopolymerization initiators, such as 2,4,6-trimethylbenzoyldiphenylphosphine oxide and bis(2,4,6-trimethylbenzoyl)-phenylphosphine oxide; benzoin-based photopolymerization initiators, such as methyl o-benzoylbenzoate, methyl benzoyl formate, benzoin methyl ether, benzoin ethyl ether, benzoin isopropyl ether, benzoin isobutyl ether, and benzoin-n-butyl ether; phenone-based photopolymerization initiators, such as 2-hydroxy-2-methyl-1-phenylpropan-1-one, p-isopropyl-α-hydroxyisobutylphenone, p-t-butyltrichloroacetophenone, 2,2-dimethoxy-2-phenylacetophenone, α,α-dichloro-4-phenoxyacetophenone, and N,N-tetraethyl-4,4-diaminobenzophenone; 1-hydroxycyclohexyl phenyl ketone; 1-phenyl-1,2-propanedione-2-(o-ethoxycarbonyl)oxime; thioxanthone-based photopolymerization initiators, such as 2-chlorothioxanthone and 2-methylthioxanthone; dibenzosuberone; 2-ethylanthraquinone; benzophenone acrylate; benzophenone; and benzil. Those photopolymerization initiators may be used alone or in combination thereof. In addition, a photosensitizer may be used in combination with the photopolymerization initiator. The blending ratio of such photopolymerization initiator and photosensitizer is preferably from 0.001 part by weight to 3 parts by weight, more preferably from 0.01 part by weight to 2 parts by weight with respect to 100 parts by weight of the monomer components in the reactive mixture.

Meanwhile, examples of the thermal polymerization initiator to be used in the polymerization through heating (thermal polymerization) include 2,2'-azobisisobutyronitrile, 2,2'-azobis(2,4-dimethylvaleronitrile), benzoyl peroxide, t-butyl hydroperoxide, cumene hydroperoxide, lauroyl peroxide, t-butyl peroxyhexanoate, and 3,5,5-trimethylhexanoyl peroxide. Those thermal polymerization initiators may be used alone or in combination thereof. The blending ratio of the thermal polymerization initiator is preferably from 0.001 part by weight to 2 parts by weight, more preferably from 0.01 part by weight to 1 part by weight with respect to 100 parts by weight of the monomer components in the reactive mixture.

Any appropriate nonpolymerizable compound may be added as the additive in accordance with purposes. Specific examples thereof include a nonpolymerizable color additive, a nonpolymerizable UV absorber, a surfactant, a refrigerant, and a thickening agent. It is not necessarily required to add such additive to the reactive mixture, and the additive may be added to the photochromic polymer, which has been obtained by the polymerization, by causing the additive to permeate into the polymer.

Examples of the nonpolymerizable color additive include 1,4-bis[(4-methylphenyl)amino]-9,10-anthraquinone (D&C Green No. 6), 1-[[4-(phenylazo)phenyl]azo]-2-naphthalenol (D&C Red No. 17), 1-hydroxy-4-[(4-methylphenyl)amino]-9,10-anthraquinone (D&C Violet No. 2), 2-(2-quinolyl)-1,3-indanedione (D&C Yellow No. 11), 4-[(2,4-dimethylphenyl)azo]-2,4-dihydro-5-methyl-2-phenyl-3H-pyrazol-3-one (C.I. Solvent Yellow 18), and 2-(1,3-dioxo-2-indanyl)-3-hydroxyquinoline (MACROLEX (trademark) Yellow-G). In addition, the examples include: a dye described in JP 2006-291006 A; an oil-soluble dye, such as C.I. Solvent Yellow or C.I. Solvent Orange described in the color index (C.I.); a disperse dye, such as C.I. Disperse Yellow or C.I. Disperse Orange described therein; and a vat-based dye. The blending amount of the nonpolymerizable color additive is preferably set within the range of from 0.001 part by weight to 0.1 part by weight with respect to 100 parts by weight of the monomer components.

Examples of the nonpolymerizable UV absorber include: benzophenones, such as 2-hydroxy-4-methoxybenzophenone and 2-hydroxy-4-octoxybenzophenone; benzotriazoles, such as 2-(2'-hydroxy-5'-methylphenyl)benzotriazole, 5-chloro-2-(3'-t-butyl-2'-hydroxy-5'-methylphenyl)benzotriazole, 2-(2'-hydroxy-5'-methylphenyl)benzotriazole, and 2-(5-chloro-2H-benzotriazol-2-yl)-6-(1,1-dimethyl)-4-methylphenol; salicylic acid derivatives; and hydroxyacetophenone

derivatives. The blending amount of the nonpolymerizable UV absorber is preferably set within the range of from 0.01 part by weight to 3 parts by weight with respect to 100 parts by weight of the monomer components.

The polymerization of the monomer components is performed under, for example, a state in which the reactive mixture is filled into a casting mold corresponding to a desired ophthalmic medical device shape. Thus, a photochromic polymer (consequently, an ophthalmic medical device) having the desired shape can be directly obtained. Alternatively, the polymerization may be performed so that a rod-shaped photochromic polymer may be obtained. An ophthalmic medical device having the desired shape can be obtained by subjecting the rod-shaped photochromic polymer to lathing.

A heating temperature at the time of the polymerization reaction performed by the heating is preferably from 50°C to 110°C, more preferably from 80°C to 100°C. In addition, a heating time at that time is preferably from 2 minutes to 60 minutes, more preferably from 10 minutes to 45 minutes. When the heating is performed under such conditions, a polymerization time can be shortened and the amount of a residual monomer can be reduced while the volatilization of the respective monomers and the deformation of the casting mold are suppressed.

Light irradiation conditions at the time of the polymerization reaction performed by the light irradiation are appropriately selected in accordance with, for example, the characteristics of the photochromic monomer and a target contact lens function. For example, the wavelength region of the light to be applied to the reactive mixture is preferably from 385 nm to 550 nm, more preferably from 400 nm to 500 nm. A light illuminance is preferably from 0.1 $mW/cm^2$ to 170 $mW/cm^2$, more preferably from 0.2 $mW/cm^2$ to 150 $mW/cm^2$. The values are each a value measured with an illuminance meter having a sensor for light having a wavelength of 405 nm, and light beams having different illuminances may be applied in a stepwise manner. An irradiation time is preferably 1 minute or more. When such light illuminance and irradiation time are adopted, even in the case where part of the light is absorbed by the photochromic monomer, the reactive mixture can be sufficiently cured. Various light sources may be applied as light sources as long as the light sources are each a light source configured to emit light having a predetermined wavelength, such as a fluorescent lamp or a LED. A plurality of light sources may be selected, and their light beams may be individually applied in a stepwise manner, or may be simultaneously applied.

The rod-shaped photochromic polymer may be obtained by, for example, loading the reactive mixture into a test tube produced from glass or the like, tightly stopping the tube, and heating the tube in a thermobath or an oven to a predetermined temperature to cure the contents. A heating temperature is preferably from 30°C to 60°C, more preferably from 35°C to 50°C. In addition, a heating time is preferably from 3 hours to 60 hours, more preferably from 5 hours to 48 hours. The heating may be performed in a stepwise manner. Further, as post-curing, heating may be performed at a temperature in the range of from 50°C to 120°C for from 2 hours to 10 hours.

In one embodiment, thermal polymerization and photopolymerization are performed in combination. It is preferred that the photopolymerization be performed after the thermal polymerization, or the thermal polymerization and the photopolymerization be simultaneously progressed by irradiating the reactive mixture with light while heating the mixture. When the thermal polymerization and the photopolymerization are performed in parallel, or the thermal polymerization is caused to precede the photopolymerization, a high-quality photochromic polymer suppressed from yellowing can be obtained.

C. Method of producing Ophthalmic Medical Device

A method of producing the above-mentioned ophthalmic medical device includes polymerizing a reactive mixture containing the above-mentioned monomer components to provide a photochromic polymer. The polymerization may include both of thermal polymerization and photopolymerization. Specifically, the polymerization may be performed as follows: the thermal polymerization and the photopolymerization are performed in parallel, or the thermal polymerization is caused to precede the photopolymerization. The monomer components, the reactive mixture, and a method of polymerizing the mixture are as described in the section B. In one embodiment, the reactive mixture is free of an organic solvent or contains the organic solvent at a content ratio of 10 parts by weight or less with respect to 100 parts by weight of the monomer components. In one embodiment, the monomer components include a photochromic monomer and a lactam ring-containing monomer, and the content ratio of the lactam ring-containing monomer in the monomer components is from 10 wt% to 50 wt%. In addition, the content ratio of a hydroxyl group-containing monomer in the monomer components may be 20 wt% or less.

The photochromic polymer obtained by the polymerization is subjected to machining, such as cutting or polishing, as required after having been removed from a casting mold or after having been removed as a rod-shaped photochromic polymer. The machining may be performed over the entirety of one surface, or each of both the surfaces, of the photochromic polymer, or may be performed on part of one surface, or each of both the surfaces, of the photochromic polymer.

From the viewpoint of reducing the amount of an undesired residue, such as an unreacted monomer, to the extent possible, the treatment of eluting the residue may be performed as required. Specifically, the treatment of eluting the residue may be performed by immersing the resultant photochromic polymer in water or an organic solvent, or a mixed solution thereof, preferably by repeating the immersion.

Further, surface modification treatment, such as low-temperature plasma treatment, atmospheric-pressure plasma treatment, or corona discharge treatment, may be performed for improving the surface characteristics of the photochromic polymer.

In addition, further, when the ophthalmic medical device is an intraocular lens, the lens may be a three-piece type lens obtained by post-fixing two support portions each formed of an elastic wire to a soft or hard optical portion material, or a one-piece type and foldable lens obtained by integrally forming an optical portion and a support portion from the same material. The polymerization may be performed in a casting mold, or the material obtained after the polymerization may be processed into a desired shape by cutting. That is, the lens removed from the casting mold may be used as it is, or may be subjected to partial processing, such as the drilling of holes for fixing the support portions. Further, the lens may be subjected to elution treatment or surface treatment as required.

D. Physical Properties of Ophthalmic Medical Device

The ophthalmic medical device may have appropriate physical properties in accordance with, for example, a desired embodiment. Physical properties suitable for a typical embodiment of the ophthalmic medical device are described below.

D-1. Hydrogel Soft Contact Lens

The water content of a hydrogel soft contact lens is preferably from 25% to 80%, more preferably from 30% to 75%. The setting of the water content to 25% or more can improve the water wettability of the surface of the lens. When the water content is more than 80%, the lens is so soft as to be incapable of retaining its shape, and when the water content is less than 25%, concern is raised in that the hydrophobicity of the surface of the lens becomes stronger, and hence the lens adsorbs to a cornea.

The hydrogel soft contact lens preferably has a Dk value of from 25 barrer to 160 barrer, and more preferably has a Dk value of from 40 barrer to 160 barrer. The Dk value is a numerical value obtained by multiplying the value of an oxygen permeability coefficient [unit: $(cm^2/sec)\cdot(mLO_2/(mL.mmHg))$] by $10^{-11}$, and the following relationship is valid: 1 barrer=$1\times10^{-11}$ $(cm^2/sec)\cdot(mLO_2/(mL\cdot mmHg))$ . A contact lens having a Dk value in the ranges is excellent in oxygen permeability, and as a result, can provide an excellent wearing feeling.

The Young's modulus (20°C) of the hydrogel soft contact lens is preferably from 0.2 MPa to 2.0 MPa, more preferably from 0.3 MPa to 1.5 MPa. When the Young's modulus falls within the ranges, the lens is excellent in wearing feeling. In addition, the lens is excellent in shape retentivity on a finger, and is hence easy to handle. When the Young's modulus is more than 2.0 MPa, the lens itself is rigid, and hence there is a risk in that the lens has a poor wearing feeling and strongly shows a foreign-body sensation. Meanwhile, when the Young's modulus is less than 0.2 MPa, the shape retentivity of the lens is poor. In addition, the risk that the lens deflects or wrinkles at the time of its wearing becomes higher.

D-2. Oxygen-permeable Hard Contact Lens

An oxygen-permeable hard contact lens preferably has a Dk value of from 130 barrer to 250 barrer. An ophthalmic medical device having a Dk value in the range is excellent in oxygen permeability, and as a result, can provide an excellent wearing feeling.

The water absorption ratio of the oxygen-permeable hard contact lens is preferably 1.0 wt% or less. A water absorption ratio of more than 1.0 wt% tends to lead to a reduction in shape stability of the lens. The water absorption ratio may be evaluated in accordance with JP 2005-181730 A.

D-3. Non-hydrogel Soft Contact Lens

A non-hydrogel soft contact lens preferably has a Dk value of from 50 barrer to 200 barrer. A contact lens having a Dk value in the range is excellent in oxygen permeability, and as a result, can provide an excellent wearing feeling.

The shape recovery ratio of the non-hydrogel soft contact lens is preferably 25% or less. A shape recovery ratio of more than 25% may lead to a feeling of wrongness or the destabilization of vision at the time of the wearing of the ophthalmic medical device. The shape recovery ratio may be evaluated in accordance with WO 00/70388 A1.

The water content of the non-hydrogel soft contact lens is, for example, less than 10 wt%, preferably 9.9 wt% or less.

D-4. Intraocular Lens

The elongation of an intraocular lens is preferably from 170% to 600%. When the elongation is less than 170%,

the lens is poor in softness, and hence it becomes difficult to fold the lens and to insert the lens from a small incised wound. An elongation of more than 600% is not preferred from the viewpoint of the shape recoverability of the lens.

The water absorption ratio of the intraocular lens is preferably from 1.5 wt% to 4.5 wt%. When the water absorption ratio is less than 1.5 wt%, glistening is liable to occur, and hence the risk that the transparency of the lens is impaired becomes higher. When the water absorption ratio is more than 4.5 wt%, there arises a risk in that the softness thereof is so high that an inconvenience occurs at the time of its insertion. The elongation and the water absorption ratio may be evaluated in accordance with WO 2018/021455 A1.

E. Optical Characteristics of Ophthalmic Medical Device

An ophthalmic medical device in one embodiment of the present invention includes a photochromic polymer, which has a repeating unit derived from a photochromic monomer and is capable of changing from an inactive state to an activated state having a visible light transmittance lower than that of the inactive state through absorption of light energy. The ophthalmic medical device has a spectral transmittance of more than 90%, preferably 92% or more, more preferably 95% or more in at least part of wavelengths in a wavelength region of less than 700 nm under the inactive state. The spectral transmittance is a value at a cornea temperature, and the same holds true for the other optical characteristics in this section.

The luminous transmittance of the ophthalmic medical device in the inactive state in a wavelength region of from 380 nm to 780 nm may be a value of preferably 75% or more, more preferably 80% or more, still more preferably 90% or more, still more preferably more than 90%.

Meanwhile, the ophthalmic medical device may have a spectral transmittance of less than 70% in at least part of wavelengths in a visible light region under the activated state, and has a spectral transmittance of, for example, 15% or more and less than 70%, preferably from 20% to 65%, more preferably from 25% to 60%.

Specifically, the ophthalmic medical device may have a spectral transmittance of less than 70% in at least part of wavelengths in a wavelength region of from 500 nm to 700 nm under the activated state, and has a spectral transmittance of, for example, 15% or more and less than 70%, preferably from 20% to 65%, more preferably from 25% to 65%.

More specifically, the ophthalmic medical device may have a spectral transmittance of less than 70% in at least part of wavelengths in a wavelength region of from 530 nm to 670 nm under the activated state, and has a spectral transmittance of, for example, 15% or more and less than 70%, preferably from 20% to 65%, more preferably from 25% to 65%.

While the ophthalmic medical device has a low light transmittance in a region where visual sensitivity is high (wavelength region of from 500 nm to 700 nm) under the activated state, the device has a high luminous transmittance over the entirety of the visible light region under the inactive state. Thus, the device can satisfactorily alleviate glare under an environment where sunshine or lighting is strong, and can secure a satisfactory field of view under an environment having moderate brightness.

In one embodiment, the ophthalmic medical device may have a spectral transmittance of 80% or less in a blue light region under the activated state, and preferably has a spectral transmittance of 70% or less. Thus, a stress on an eye resulting from blue light can be alleviated. The phrase "has a spectral transmittance of 80% or less in the blue light region" means that the maximum value of the spectral transmittance in the blue light region is 80% or less.

In one embodiment, a value obtained by multiplying the luminous transmittance of the ophthalmic medical device in the inactive state or the activated state by 0.2 is smaller than the minimum spectral transmittance thereof in each state in a wavelength region of from 500 nm to 650 nm. When such relationship is satisfied, a person can drive a vehicle, such as an automobile, without any particular hitch while wearing the ophthalmic medical device.

In one embodiment, the relative visual attenuation coefficient (Q-value) of the ophthalmic medical device in the activated state with respect to a red signal is at least 0.8, the Q-value thereof with respect to a yellow signal is at least 0.6, the Q-value thereof with respect to a green signal is at least 0.6, and the Q-value thereof with respect to a blue signal is at least 0.4. When the ophthalmic medical device has such Q-values, a person can drive a vehicle, such as an automobile, without any particular hitch while wearing the ophthalmic medical device. The relative visual attenuation coefficients (Q-values) are each a value defined in JIS T 7333.

In one embodiment, the ophthalmic medical device may change from the inactive state to the activated state within 1 minute from a start of irradiation with light having an illuminance of 50,000 lux from a xenon lamp.

Examples

Now, the present invention is specifically described by way of Examples. However, the present invention is not limited by these Examples. Measurement methods and evaluation methods for characteristics are as described below. Even when the irradiation of each of ophthalmic medical devices produced in Experimental Examples 1 to 13 and C1 to C3 with light under the following irradiation conditions was continued for 1 minute or more, the light transmittance

thereof did not change any more: the light was LED light having a wavelength of 405 nm and an illuminance of 10 $mW/cm^2$ or more. Accordingly, a state in which the ophthalmic medical device was irradiated with the light having the predetermined wavelength (LED light having a wavelength of 405 nm and an illuminance of 10 $mW/cm^2$ or more) for 1 minute or more was judged as an "activated state." In addition, a change in light transmittance after the stop of the irradiation was observed, and a state in which the device was left at rest for 1 hour or more after the stop of the irradiation with the light having the predetermined wavelength (LED light having a wavelength of 405 nm and an illuminance of 10 $mW/cm^2$ or more) was judged as an "inactive state."

«Transparency»

The appearance of a contact lens was visually observed, and was evaluated on the basis of the following evaluation criteria.

(Evaluation Criteria)

A: The contact lens is free of fogging and is extremely excellent in transparency, and is hence optimum as a contact lens.
B: The contact lens slightly shows fogging, but has transparency causing no problem as a contact lens.
C: The contact lens is observed to become clouded and is poor in transparency, and hence it is difficult to use the lens as a contact lens.
D: The contact lens remarkably becomes clouded and is extremely poor in transparency, and hence it is impossible to use the lens as a contact lens.

<<Water Content>>

The weight (W (g)) of a lens brought into an equilibrium water-containing state in saline at 20°C was measured, and the weight ($W_0$ (g)) of the lens after such hydration treatment in the state of being dried in a dryer at 105°C was measured. The water content (wt%) thereof was calculated by using those measured values $W_0$ and W in accordance with the following equation.
Water content

$$(wt\%)=\{(W-W_0)/W\}\times 100$$

<<Oxygen Permeability Coefficient (Dk)>>

The oxygen permeability coefficient of a lens was measured with an IPI type film oxygen permeability meter (manufactured by Rikaseiki Co., Ltd.) in saline at 35°C. The measurement was performed by using the following lenses having different thicknesses as test samples: a lens having a center thickness of about 0.1 mm was used alone, or a lens obtained by superimposing the two to four lenses was used. At the time of the calculation of the value, the calculation was performed in conformity with ISO 18369-4 (2006) in consideration of an edge effect. Menicon 2 WEEK PremiO (manufactured by Menicon Co., Ltd.) was used as a reference standard, and its Dk value was normalized to 129. The oxygen permeability coefficient is represented in the unit of ($\times 10^{-11}$ ($cm^2$/sec)·($mLO_2$/mL$\times$mmHg)).

<<Tensile Test>>

A dumbbell-shaped sample whose extension portion had a width of about 1.8 mm and a thickness of about 0.1 mm was punched out of a produced contact lens, and was subjected to a tensile test. The measurement was performed with a Shimadzu precision universal tester "AUTOGRAPH AG-IS TYPE MS" (manufactured by Shimadzu Corporation), and the tensile elastic modulus (MPa) of the sample was calculated as a Young’s modulus from the stress-elongation curve thereof. In addition, the tensile strength at break (MPa) and tensile elongation at break (%) thereof were also read from the strength and elongation thereof at the time of its rupture. All the measurements were performed in saline regulated to 20°C, and a tensile rate was set to 10 mm/min.

<<Light Transmittance Measurement>>

(Activated State)

A lens was subjected to conditioning for about 1 hour in a thermobath whose temperature had been controlled to 40°C in advance. The lens was loaded into a cell together with distilled water whose temperature had been controlled to 40°C under a state in which the lens was set in a jig for lens measurement (manufactured by Menicon Co., Ltd.), and LED light having a wavelength of from 385 nm to 405 nm (LED1801, sales agency: Beauty World Co., Ltd.) was applied to the lens for about 1 minute so that the light was applied from the outside of the cell to the lens. Simultaneously with the application, a temperature gauge was used to recognize that the temperature of the distilled water in the cell was 35°C±2°C. After that, the transmittance (T%) of the lens was immediately measured with a spectrophotometer (UV-2550, manufactured by Shimadzu Corporation) (measurement condition: high speed, sampling pitch: 2 nm). The illuminance of the LED light applied to the cell (measured at a wavelength of 405 nm with UVD-150 and UVD-S405 manufactured by Ushio Inc.) was 10 mW/cm$^2$ or more. Transmittance measurement in a wavelength region of from 280 nm to 380 nm and that in a wavelength region of from 380 nm to 780 nm were separately performed in order to reduce an influence of color fading.

(Inactive State)

Measurement was performed in the same manner as that described above except that: the lens was subjected to temperature control in the same manner as that described above for 1 hour or more under a light-shielded state; and the lens was loaded into the cell, and then the measurement was immediately performed.

[Used Components]

Components used in Experimental Examples 1 to 13 and C1 to C3 are described below together with their abbreviations.

- Macromonomer (A): A prepolymer obtained by polymerizing methyl methacrylate, 2-hydroxybutyl methacrylate, allyl methacrylate, or ethylene glycol dimethacrylate
- Macromonomer (B): A siloxane-containing macromonomer represented by the following formula (X), which is obtained by causing carbinol-modified polydimethylsiloxane in which the number of repetitions of a dimethylsiloxane unit is about 40, isophorone diisocyanate, and 2-hydroxyethyl acrylate to react with each other (average of "n" in the formula: about 40)

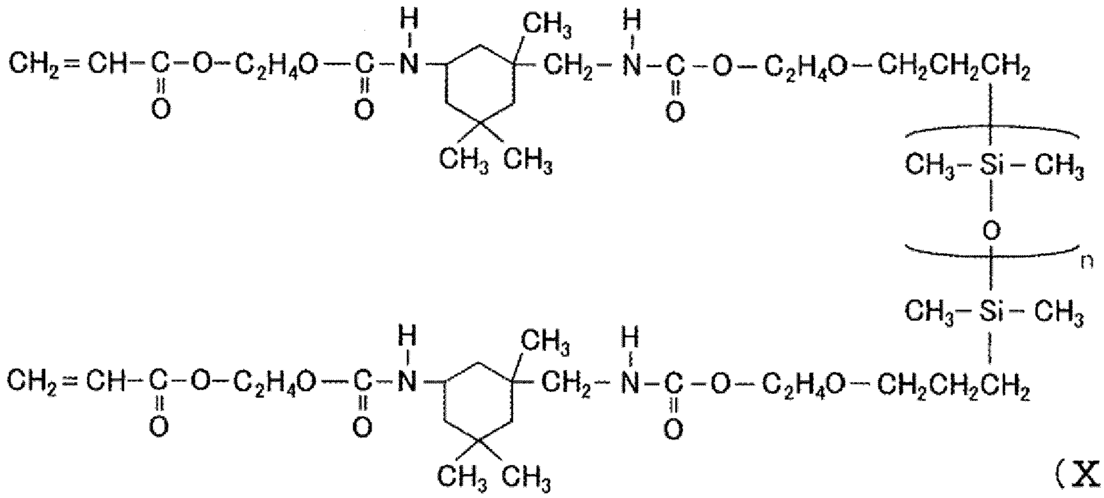

(X)

- TRIS: Tris(trimethylsiloxy)silylpropyl methacrylate
- MMA: Methyl methacrylate
- 2-MTA: 2-Methoxyethyl acrylate
- N-VP: N-Vinyl-2-pyrrolidone
- DMAA: N,N-Dimethylacrylamide
- N-MMP: 1-Methyl-3-methylene-2-pyrrolidinone

- HEMA: 2-Hydroxyethyl methacrylate
- GMA: Glycerol methacrylate
- EDMA: Ethylene glycol dimethacrylate
- AMA: Allyl methacrylate
- RB246: 1,4-Bis(4-(2-methacryloxyethyl)phenylamino) anthraquinone
- V-65: 2,2'-Azobis(2,4-dimethylvaleronitrile)
- Irg819: Phenylbis(2,4,6-trimethylbenzoyl)phosphine oxide
- PC01: An indenonaphthopyran-based polymerizable photochromic compound represented by the following formula, which is prepared in accordance with the above-mentioned synthesis method

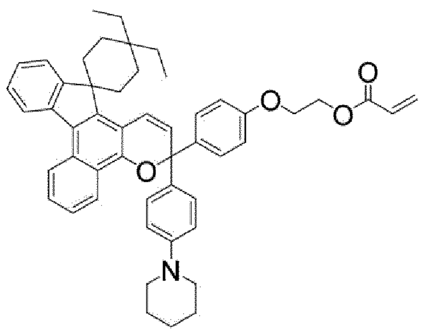

(PC01)

- PC02: An indenonaphthopyran-based polymerizable photochromic compound represented by the following formula, which is prepared in accordance with the above-mentioned synthesis method

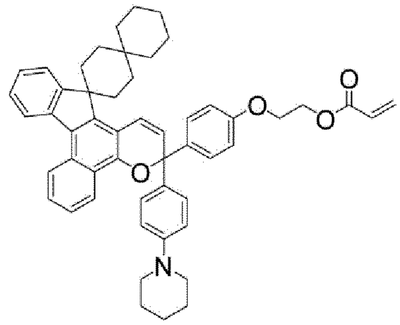

(PC02)

- ABCH: A fulgide-based nonpolymerizable photochromic compound shown below

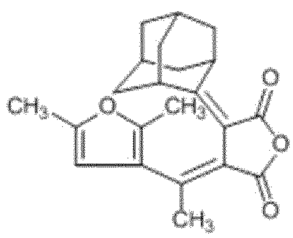

[Experimental Example 1]

Respective components were mixed so that blending ratios (part(s) by weight) shown in Table 1 were obtained. Thus, a reactive mixture was prepared. The resultant reactive mixture was poured into a casting mold having a contact lens shape (made of polypropylene, corresponding to a contact lens having a diameter of about 14 mm and a thickness of about 0.1 mm). Next, the casting mold was loaded into a heat circulation-type soaking dryer (manufactured by Taitec Corporation) set at 90°C, and was left at rest for 30 minutes so that the reactive mixture was cured. The resultant polymer material (dry lens) was removed from the casting mold, and was then subjected to low-pressure plasma treatment in a carbon dioxide atmosphere at 25 W for 3 minutes. Next, the treated material was subjected to hydration treatment by being immersed in saline to be caused to absorb the water. Thus, a contact lens was obtained.

[Experimental Examples 2 to 9 and 11 to 13, and C1 to C3]

Respective components were mixed so that blending ratios shown in Table 1 were obtained. Thus, a reactive mixture was prepared. The resultant reactive mixture was poured into a casting mold having a contact lens shape (made of polypropylene, corresponding to a contact lens having a diameter of about 14 mm and a thickness of about 0.1 mm). Next, light from a LED lamp was applied to the casting mold to cure the reactive mixture. The application was performed in two stages, and the light was applied at an illuminance of about 1 $mW/cm^2$ for 15 minutes in the first stage, while the light was applied at an illuminance of about 20 $mW/cm^2$ for 5 minutes in the second stage (in each stage, the illuminance was measured at 405 nm). The resultant polymer material (dry lens) was subjected to the same plasma treatment and hydration treatment as those of Experimental Example 1. Thus, contact lenses were obtained.

[Experimental Example 10]

Respective components were mixed so that blending ratios shown in Table 1 were obtained. Thus, a reactive mixture was prepared. The resultant reactive mixture was poured into a casting mold having a contact lens shape (made of polypropylene, corresponding to a contact lens having a diameter of about 14 mm and a thickness of about 0.1 mm). The casting mold was loaded into a heat circulation-type soaking dryer (manufactured by Taitec Corporation) set at 90°C, and was left at rest for 30 minutes, followed by the application of light from a LED lamp. Thus, the reactive mixture was cured. The application of light was performed in two stages, and the light was applied at an illuminance of about 1 $mW/cm^2$ for 15 minutes in the first stage, while the light was applied at an illuminance of about 20 $mW/cm^2$ for 5 minutes in the second stage (in each stage, the illuminance was measured at 405 nm). The resultant polymer material (dry lens) was subjected to the same plasma treatment and hydration treatment as those of Experimental Example 1. Thus, a contact lens was obtained.

Table 1

| | | Experimental Example | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | C1 | C2 | C3 |
| Silicone-containing monomer | Macromonomer (B) | - | 5.0 | 30.1 | 29.0 | 34.0 | 5.0 | 7.0 | 10.0 | 10.0 | 5.0 | 4.5 | 4.5 | 4.0 | 5.0 | 29.0 | - |
| | TRIS | - | 26.0 | 24.5 | 24.6 | 25.0 | 29.0 | 27.0 | 20.5 | 20.0 | 25.0 | 27.0 | 26.0 | 25.0 | 30.0 | 19.0 | - |
| Hydrophobic monomer | Macromonomer (A) | 20.0 | | - | - | - | - | - | - | - | - | - | - | - | - | - | - |
| | MMA | 16.0 | - | - | - | - | - | - | - | - | - | - | - | - | - | - | - |
| | 2-MTA | - | 28.7 | - | - | - | 21.7 | 20.7 | 19.2 | 19.2 | 27.7 | 23.3 | 21.4 | 20.4 | 24.7 | 19.7 | - |
| Lactam ring-containing monomer | N-VP | 15.7 | 39.0 | - | - | - | 30.0 | 30.0 | 40.0 | 39.0 | 41.0 | 36.5 | 34.0 | 32.5 | 40.0 | 8.0 | - |
| | N-MMP | - | - | 34.0 | 35.0 | 19.5 | - | - | - | - | - | - | - | - | - | - | - |
| Hydrophilic monomer | DMAA | 47.0 | - | 10.0 | 10.0 | 20.0 | 13.0 | 14.0 | 9.0 | 10.5 | - | - | - | - | - | - | - |
| | HEMA | - | - | - | - | - | - | - | - | - | - | - | 13.0 | 17.0 | - | 23.0 | 59.0 |
| | GMA | - | - | - | - | - | - | - | - | - | - | 7.5 | - | - | - | - | 39.4 |
| Cross-linkable monomer | EDMA | 0.3 | - | 0.4 | 0.4 | 0.5 | - | - | - | - | - | - | - | - | - | - | 0.6 |
| | AMA | - | 0.3 | - | - | - | 0.3 | 0.3 | 0.3 | 0.3 | 0.3 | 0.3 | 0.3 | 0.2 | 0.3 | 0.3 | - |
| Polymerizable color additive | RB246 | - | - | 0.01 | - | - | - | - | - | 0.01 | - | - | - | - | - | - | - |
| Photo chromic monomer | PC01 | - | 1.0 | 1.0 | - | - | 1.0 | 1.0 | 1.0 | 1.0 | 1.0 | 0.9 | 0.9 | 0.8 | - | 1.0 | - |
| | PC02 | 1.0 | - | - | 1.0 | 1.0 | - | - | - | - | - | - | - | - | - | - | 1.0 |
| Initiator | Irg819 | - | 0.6 | 0.5 | 0.5 | 0.6 | 0.6 | 0.6 | 0.6 | 0.6 | 0.6 | 0.6 | 0.6 | 0.6 | 0.6 | 0.6 | 0.5 |
| | V-65 | 0.2 | - | - | - | - | - | - | - | - | 0.2 | - | - | - | - | - | - |
| Nonpolymerizable photo chromic compound | ABCH | - | - | - | - | - | - | - | - | - | - | - | - | - | 1.0 | - | - |

The various characteristics of the contact lenses obtained in Experimental Examples described above were evaluated, and the light transmittances thereof in their activated states and inactive states in a wavelength region of from 380 nm to 780 nm were measured. The results are shown in Table 2 and Table 3 (a maximum value and a minimum value shown in Table 3 represent the maximum value and minimum value of spectral transmittances in each wavelength region, respectively). In addition, the light transmittance spectra of the contact lenses obtained in Experimental Examples 1 to 3 and C1 are shown in FIGS. 1. The characteristics of the contact lenses obtained in Experimental Examples C2 and C3 except their transparencies were not evaluated because the lenses became clouded.

Table 2

| | Experimental Example | | | | | | | | | | | | | | | |
|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | C1 | C2 | C3 |
| Water content (%) | 71 | 56 | 44 | 44 | 34 | 52 | 52 | 56 | 56 | 53 | 51 | 49 | 50 | 59 | - | - |
| Dk (barrer) | 50 | 59 | 131 | 129 | 121 | 70 | 76 | 79 | 70 | 75 | 72 | 76 | 70 | 55 | - | - |
| Tensile strength at break (MPa) | 1.32 | 1.48 | 1.64 | 1.84 | 1.16 | 1.39 | 1.73 | 0.87 | 1.30 | 1.50 | 0.95 | 0.86 | 0.58 | 1.10 | - | - |
| Tensile elongation at break (%) | 121 | 323 | 203 | 202 | 108 | 334 | 354 | 206 | 272 | 324 | 271 | 301 | 225 | 295 | - | - |
| Tensile elastic modulus (MPa) | 1.74 | 0.32 | 1.02 | 1.13 | 1.57 | 0.28 | 0.30 | 0.40 | 0.46 | 0.33 | 0.35 | 0.27 | 0.24 | 0.30 | - | - |
| Transparency | A | A | A | A | A | A | A | A | A | A | A | A | A | A | C | D |
| Time required for contact lens to activate from start of application of LED light having wavelength of from 385 nm to 405 nm | <1 min | <1 min | <1 min | <1 min | <1 min | <1 min | <1 min | <1 min | <1 min | <1 min | <1 min | <1 min | <1 min | <1 min | <1 min | <1 min |

Table 3

| | | | | Experimental Example | | | | | | | | | | | | | | | |
|---|---|---|---|---|---|---|---|---|---|---|---|---|---|---|---|---|---|---|---|
| | Wavelength region | Measurement item | | 1 | 2 | 3 | 4 | 5 | 6 | 7 | 8 | 9 | 10 | 11 | 12 | 13 | C1 | C2 | C3 |
| Inactive state | Less than 700 nm | Spectral transmittance (%) | Maximum value | 97.7 | 96.0 | 97.0 | 93.0 | 95.8 | 97.2 | 97.3 | 97.8 | 100 | 97.0 | 94.4 | 95.3 | 93.7 | 99.0 | - | - |
| | Visible light region (380 nm to 780 nm) | Spectral transmittance (%) | Maximum value | 100 | 98.2 | 98.2 | 94.9 | 97.7 | 100 | 100 | 98.5 | 100 | 98.1 | 96.8 | 98.2 | 96.0 | 99.1 | | |
| | | | Minimum value | 49.6 | 26.7 | 30.8 | 27.5 | 22.8 | 33.5 | 36.5 | 33.6 | 32.9 | 41.3 | 31.1 | 39.8 | 25.3 | 54.0 | - | - |
| | Visible light region (380 nm to 780 nm) | Spectral transmittance (%) | Average | 92.3 | 88.3 | 88.9 | 83.9 | 85.3 | 90.8 | 91.2 | 90.6 | 92.6 | 91.2 | 87.1 | 88.8 | 85.5 | 94.1 | - | - |
| Activated state | Visible light region (380 nm to 780 nm) | Spectral transmittance (%) | Maximum value | 99.5 | 98.2 | 98.2 | 94.9 | 97.7 | 96.3 | 97.0 | 95.0 | 100 | 97.7 | 93.5 | 92.2 | 91.1 | 99.1 | | |
| | | | Minimum value | 27.0 | 18.8 | 4.1 | 13.4 | 11.2 | 20.5 | 22.9 | 20.6 | 22.7 | 29.9 | 24.6 | 20.6 | 16.1 | 65.0 | | |
| | 500 nm to 700 nm | Spectral transmittance (%) | Maximum value | 68.7 | 72.5 | 73.5 | 75.6 | 73.2 | 74.0 | 75.3 | 72.7 | 79.1 | 82.2 | 73.8 | 69.4 | 65.9 | 99.5 | | |
| | | | Minimum value | 33.6 | 36.7 | 29.6 | 42.8 | 36.8 | 37.5 | 39.5 | 38.0 | 43.0 | 49.8 | 38.8 | 32.7 | 28.2 | 64.7 | | |
| | 530 nm to 670 nm | Spectral transmittance (%) | Maximum value | 57.1 | 58.9 | 58.1 | 62.9 | 59.0 | 60.0 | 61.4 | 58.4 | 63.4 | 68.1 | 58.9 | 53.5 | 49.1 | 99.4 | | |
| | | | Minimum value | 33.6 | 36.7 | 29.6 | 42.8 | 36.8 | 37.5 | 39.5 | 38.0 | 43.0 | 46.5 | 38.8 | 32.7 | 28.2 | 65.0 | | |
| | Blue light region (380 nm to 500 nm) | Spectral transmittance (%) | Maximum value | 67.0 | 59.3 | 54.1 | 65.2 | 62.4 | 63.9 | 66.0 | 64.1 | 68.8 | 69.0 | 63.7 | 59.6 | 54.6 | 89.3 | - | - |
| | | | Minimum value | 27.0 | 18.8 | 4.1 | 13.4 | 11.2 | 20.5 | 22.9 | 20.6 | 22.7 | 29.9 | 24.6 | 20.6 | 16.1 | 65.0 | | |

In addition, with regard to each of the contact lenses of Experimental Examples 2 and C1, the lens immediately after the polymerization was removed from the casting mold, and then the one lens was immediately immersed in a brown bottle containing 5 mL of acetonitrile. The lid of the bottle was firmly closed, and then the lens was subjected to extraction treatment in a dryer at 50°C for 5 hours. The light transmittances of the lens before and after the treatment in the activated state in a wavelength region of from 380 nm to 780 nm were measured. The results are shown in FIG. 2 and FIG. 3. The lens subjected to the same hydration treatment as that of Experimental Example 1 immediately after the removal of the lens from the casting mold immediately after the polymerization was defined as a lens before the treatment.

As shown in Table 3 and FIGS. 1, each of the contact lenses of Experimental Examples 1 to 13 had a high light transmittance under the inactive state. Meanwhile, each of the contact lenses of Experimental Examples C2 and C3 became clouded because compatibility between the monomer components was insufficient.

As shown in FIG. 2 and FIG. 3, the contact lens of Experimental Example 2 using the polymerizable photochromic compound maintained its photochromic property even after the extraction treatment, but the contact lens of Experimental Example C1 using the nonpolymerizable photochromic compound lost its photochromic property after the extraction treatment. It is found from the foregoing that the nonpolymerizable photochromic compound is liable to be eluted from the polymer material, and is hence poor in safety or stability.

In addition, the yellowing of each of the contact lenses of Experimental Examples 2 and 11 to 13, and C1 was observed, though the yellowing was in an allowable range in practical use. In contrast, in each of the lenses of Experimental Example 10 in which the polymerization was performed by using photopolymerization and thermal polymerization in combination, and the other Experimental Examples in each of which the N-vinyl lactam-based monomer and the (meth)acrylamide-based compound were used in combination, yellowing was effectively suppressed. For reference, photographs of the lenses obtained in Experimental Examples 2, 8, and 10 (after their hydration) are shown in FIG. 4.

Industrial Applicability

The present invention is suitably used in the field of a contact lens or an intraocular lens.

## Claims

1. An ophthalmic medical device, comprising a photochromic polymer obtained by polymerizing monomer components including a photochromic monomer and a lactam ring-containing monomer,

   wherein a content ratio of the lactam ring-containing monomer in the monomer components is from 10 wt% to 50 wt%, and
   wherein the ophthalmic medical device is a contact lens or an intraocular lens.

2. The ophthalmic medical device according to claim 1, wherein the lactam ring-containing monomer includes at least one kind selected from an N-vinyl lactam and a methylene lactam.

3. The ophthalmic medical device according to claim 1 or 2, wherein the monomer components further include at least one kind selected from an N,N-dialkyl (meth)acrylamide and an N,N-dialkylaminoalkyl (meth)acrylamide.

4. The ophthalmic medical device according to claim 3, wherein a total content ratio of the lactam ring-containing monomer, and the at least one kind selected from the N,N-dialkyl (meth)acrylamide and the N,N-dialkylaminoalkyl (meth)acrylamide in the monomer components is from 25 wt% to 65 wt%.

5. The ophthalmic medical device according to any one of claims 1 to 4, wherein the monomer components further include a silicone-containing monomer.

6. The ophthalmic medical device according to any one of claims 1 to 5, wherein a content ratio of the photochromic monomer in the monomer components is from 0.001 wt% to 5 wt%.

7. The ophthalmic medical device according to any one of claims 1 to 6, wherein the photochromic monomer is a T-type photochromic compound.

8. The ophthalmic medical device according to any one of claims 1 to 7, wherein the photochromic monomer is a naphthopyran compound.

**9.** The ophthalmic medical device according to any one of claims 1 to 8, wherein the photochromic monomer is represented by the following structural formula (1):

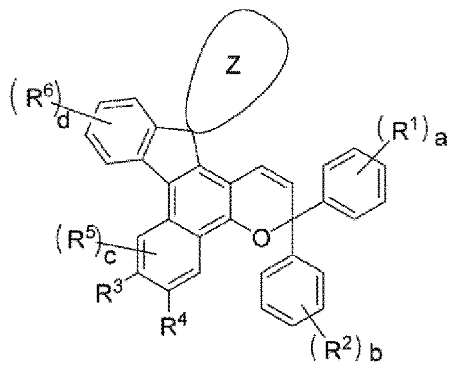

(1)

where $R^1$ and $R^2$ each independently represent a group having a radical-polymerizable group, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, or an arylthio group having 6 to 10 carbon atoms that may have a substituent,

"a" represents an integer of from 0 to 5, and "b" represents an integer of from 0 to 5, provided that a+b=1 to 10,
at least one of $R^1$ or $R^2$ represents the group having the radical-polymerizable group,
the group having the radical-polymerizable group is a group represented by the following formula (2):

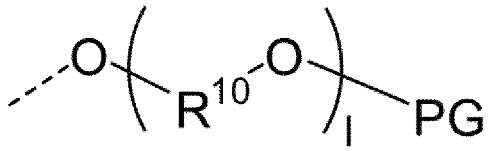

(2)

(where $R^{10}$ represents a linear or branched alkylene group having 1 to 10 carbon atoms,

"l" represents an integer of from 0 to 50, and when "l" represents 2 or more, unit groups in "l" pairs of parentheses may be groups identical to or different from each other, and
PG represents the radical-polymerizable group),
a ring Z represented by the following formula (Z), the ring being spiro-bonded to a carbon atom at a 13-position of the formula (1):

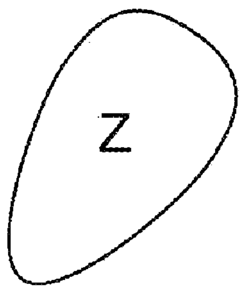

(Z)

is
an aliphatic cyclic group that may have a substituent, the group having 3 to 20 carbon atoms for forming the ring together with the carbon atom at the 13-position,
a condensed polycyclic group obtained by condensing the aliphatic cyclic group with an aromatic ring or an aromatic heterocycle that may have a substituent,
a heterocyclic group that may have a substituent, the group having 3 to 20 atoms for forming the ring together with the carbon atom at the 13-position, or

a condensed polycyclic group obtained by condensing the heterocyclic group with an aromatic ring or an aromatic heterocycle that may have a substituent,
$R^3$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a cyano group, a halogen atom, an alkylthio group having 1 to 6 carbon atoms, an arylthio group having 6 to 10 carbon atoms that may have a substituent, a nitro group, a formyl group, a hydroxycarbonyl group, an alkylcarbonyl group having 2 to 7 carbon atoms, an alkoxycarbonyl group having 2 to 7 carbon atoms, an aralkyl group having 7 to 11 carbon atoms that may have a substituent, an aralkoxy group having 7 to 11 carbon atoms that may have a substituent, an aryloxy group having 6 to 12 carbon atoms that may have a substituent, an aryl group having 6 to 12 carbon atoms that may have a substituent, a heteroaryl group having 3 to 12 carbon atoms that may have a substituent, a thiol group, an alkoxyalkylthio group having 2 to 9 carbon atoms, a haloalkylthio group having 1 to 6 carbon atoms, or a cycloalkylthio group having 3 to 8 carbon atoms,
$R^4$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, an arylthio group having 6 to 10 carbon atoms that may have a substituent, a nitro group, a formyl group, a hydroxycarbonyl group, an alkylcarbonyl group having 2 to 7 carbon atoms, an alkoxycarbonyl group having 2 to 7 carbon atoms, an aralkyl group having 7 to 11 carbon atoms that may have a substituent, an aralkoxy group having 7 to 11 carbon atoms that may have a substituent, an aryloxy group having 6 to 12 carbon atoms that may have a substituent, an aryl group having 6 to 12 carbon atoms that may have a substituent, a heteroaryl group having 3 to 12 carbon atoms that may have a substituent, a thiol group, an alkoxyalkylthio group having 2 to 9 carbon atoms, a haloalkylthio group having 1 to 6 carbon atoms, or a cycloalkylthio group having 3 to 8 carbon atoms, and
$R^3$ and $R^4$ may form a ring represented by the following formula (3) together:

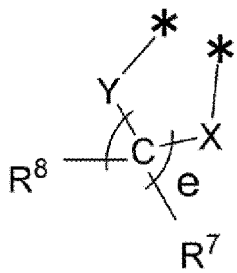

(3)

[where * represents a carbon atom at a 6-position or a 7-position thereof,
one, or each of both, of X and Y represents a sulfur atom, a methylene group, an oxygen atom, or a group represented by the following formula:

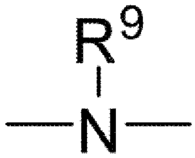

(where $R^9$ represents a hydrogen atom, a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an aryl group having 6 to 12 carbon atoms that may have a substituent, or a heteroaryl group having 3 to 12 carbon atoms that may have a substituent),

$R^7$ and $R^8$ each independently represent a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a cyano group, a nitro group, a formyl group, a hydroxycarbonyl group, an alkylcarbonyl group having 2 to 7 carbon atoms, an alkoxycarbonyl group having 2 to 7 carbon atoms, a halogen atom, an aralkyl group having 7 to 11 carbon atoms that may have a substituent, an aralkoxy group having 7 to 11 carbon atoms that may

have a substituent, an aryl group having 6 to 12 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent, and
$R^7$ and $R^8$ may form an aliphatic ring together with a carbon atom to which $R^7$ and $R^8$ are bonded, and "e" represents an integer of from 1 to 3],
$R^5$ represents a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a cyano group, a nitro group, a formyl group, a hydroxycarbonyl group, an alkylcarbonyl group having 2 to 7 carbon atoms, an alkoxycarbonyl group having 2 to
7 carbon atoms, a halogen atom, an aralkyl group having 7 to 11 carbon atoms that may have a substituent, an aralkoxy group having 7 to 11 carbon atoms that may have a substituent, an aryl group having 6 to 12 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent,
"c" represents an integer of from 0 to 2, and when "c" represents 2, $R^5$s may represent groups identical to or different from each other,
$R^6$ represents a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group, a halogen atom, an aralkyl group having 7 to 11 carbon atoms that may have a substituent, an aralkoxy group having 7 to 11 carbon atoms that may have a substituent, a thiol group, an alkylthio group having 1 to 6 carbon atoms, an alkoxyalkylthio group having 2 to 9 carbon atoms, a haloalkylthio group having 1 to 6 carbon atoms, a cycloalkylthio group having 3 to 8 carbon atoms, or an arylthio group having 6 to 10 carbon atoms that may have a substituent, and
"d" represents an integer of from 0 to 4, and when "d" represents 2 or more, $R^6$s may represent groups identical to or different from each other.

**10.** The ophthalmic medical device according to any one of claims 1 to 9, wherein the monomer components further include at least one kind selected from an alkyl (meth)acrylate, an alkoxyalkyl (meth)acrylate, and an aromatic ring-containing (meth)acrylate.

**11.** The ophthalmic medical device according to any one of claims 1 to 10, wherein a content ratio of a hydroxyl group-containing monomer in the monomer components is 20 wt% or less.

**12.** A method of producing the ophthalmic medical device of any one of claims 1 to 11, comprising polymerizing a reactive mixture, which contains monomer components including a photochromic monomer and a lactam ring-containing monomer, and is free of an organic solvent or contains the organic solvent at a content ratio of 10 parts by weight or less with respect to 100 parts by weight of the monomer components, to provide a photochromic polymer, wherein a content ratio of the lactam ring-containing monomer in the monomer components is from 10 wt% to 50 wt%.

**13.** The method according to claim 12, wherein a content ratio of a hydroxyl group-containing monomer in the monomer components is 20 wt% or less.

**14.** The method according to claim 12 or 13, wherein the polymerizing includes thermal polymerization and photopolymerization.

**15.** An ophthalmic medical device, comprising a photochromic polymer, which has a repeating unit derived from a photochromic monomer and is capable of changing from an inactive state to an activated state having a visible light transmittance lower than that of the inactive state through absorption of light energy,

wherein the ophthalmic medical device has a spectral transmittance of more than 90% in at least part of wavelengths in a wavelength region of less than 700 nm under the inactive state, and
wherein the ophthalmic medical device is a contact lens or an intraocular lens.

**16.** The ophthalmic medical device according to claim 15, wherein the ophthalmic medical device has a spectral transmittance of less than 70% in at least part of wavelengths in a visible light region under the activated state.

**17.** The ophthalmic medical device according to claim 15 or 16, wherein the ophthalmic medical device has a spectral transmittance of less than 70% in at least part of wavelengths in a wavelength region of from 500 nm to 700 nm under the activated state.

**18.** The ophthalmic medical device according to any one of claims 15 to 17, wherein the ophthalmic medical device has a spectral transmittance of less than 70% over an entirety of a wavelength region of from 530 nm to 670 nm under the activated state.

**19.** The ophthalmic medical device according to any one of claims 15 to 18, wherein the ophthalmic medical device has a spectral transmittance of 15% or more and less than 70% in at least part of wavelengths in a wavelength region of from 500 nm to 700 nm under the activated state.

**20.** The ophthalmic medical device according to any one of claims 15 to 19, wherein the ophthalmic medical device has a spectral transmittance of 15% or more and less than 70% over an entirety of a wavelength region of from 530 nm to 670 nm under the activated state.

**21.** The ophthalmic medical device according to any one of claims 15 to 20, wherein the ophthalmic medical device has a spectral transmittance of 80% or less in a blue light region under the activated state.

**22.** The ophthalmic medical device according to any one of claims 15 to 21, wherein the ophthalmic medical device has a spectral transmittance of 70% or less in a blue light region under the activated state.

**23.** The ophthalmic medical device according to any one of claims 15 to 22, wherein the ophthalmic medical device has a luminous transmittance of 75% or more in a wavelength region of from 380 nm to 780 nm under the inactive state.

**24.** The ophthalmic medical device according to any one of claims 15 to 23, wherein the ophthalmic medical device is a hydrogel lens having a Dk value of from 25 barrer to 160 barrer, having a water content of from 25 wt% to 80 wt%, and having a Young's modulus of from 0.2 MPa to 2.0 MPa.

**25.** The ophthalmic medical device according to any one of claims 15 to 23, wherein the ophthalmic medical device is an oxygen-permeable hard contact lens having a Dk value of from 130 barrer to 250 barrer and having a water absorption ratio of 1.0 wt% or less.

**26.** The ophthalmic medical device according to any one of claims 15 to 23, wherein the ophthalmic medical device is an oxygen-permeable soft contact lens having a Dk value of from 50 barrer to 200 barrer and having a shape recovery ratio of 25% or less.

**27.** The ophthalmic medical device according to any one of claims 15 to 23, wherein the ophthalmic medical device is an intraocular lens having an elongation of from 170% to 600% and having a water absorption ratio of from 1.5 wt% to 4.5 wt%.

**28.** The ophthalmic medical device according to any one of claims 15 to 27, wherein the ophthalmic medical device changes from the inactive state to the activated state within 1 minute from a start of irradiation with light having an illuminance of 50,000 lux from a xenon lamp.

FIGS. 1

(a)

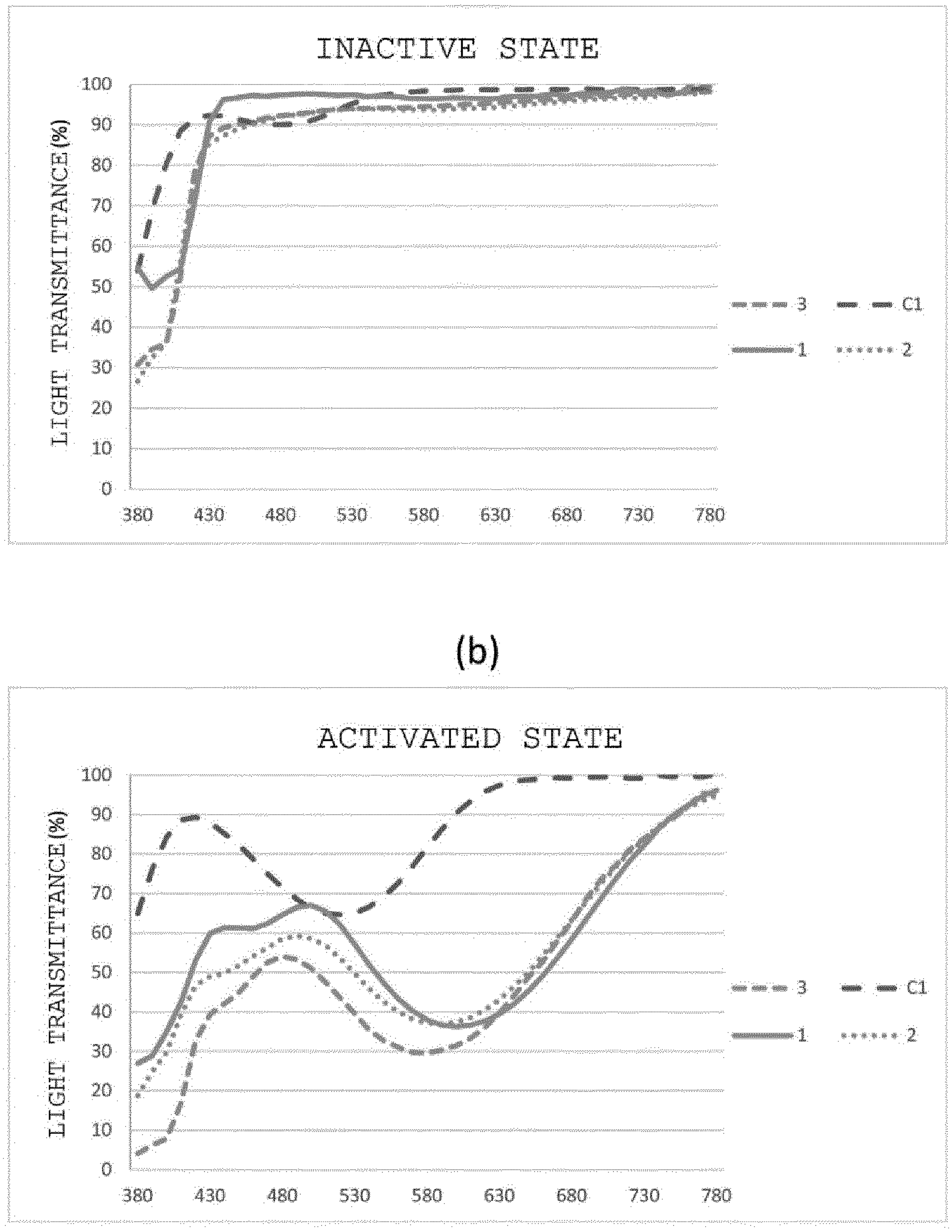
INACTIVE STATE
LIGHT TRANSMITTANCE(%)
100
90
80
70
60
50
40
30
20
10
0
380
430
480
530
580
630
680
730
780
3
C1
1
2
(b)
ACTIVATED STATE
LIGHT TRANSMITTANCE(%)
100
90
80
70
60
50
40
30
20
10
0
380
430
480
530
580
630
680
730
780
3
C1
1
2

FIG. 2

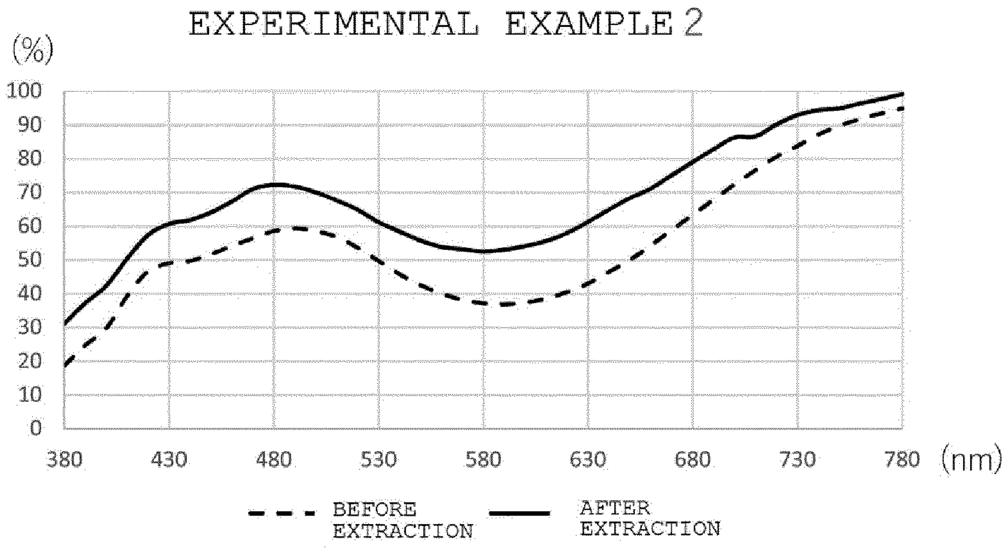
EXPERIMENTAL EXAMPLE 2
(%)
100
90
80
70
60
50
40
30
20
10
0
380
430
480
530
580
630
680
730
780
(nm)
BEFORE EXTRACTION
AFTER EXTRACTION

FIG. 3

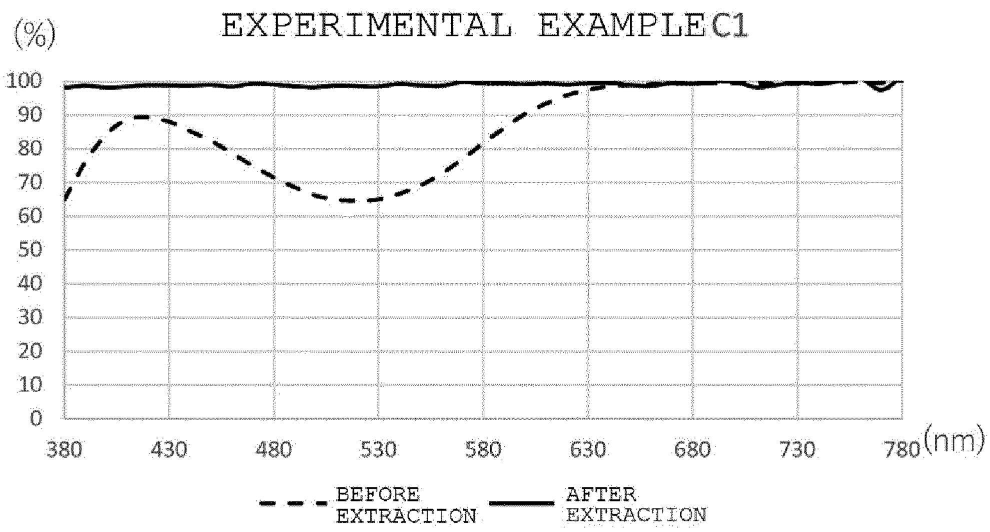
EXPERIMENTAL EXAMPLE C1
(%)
100
90
80
70
60
50
40
30
20
10
0
380
430
480
530
580
630
680
730
780
(nm)
BEFORE EXTRACTION
AFTER EXTRACTION

FIG. 4

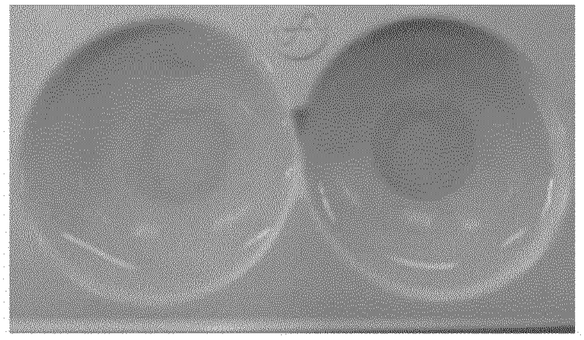

EXPERIMENTAL EXAMPLE 2 EXPERIMENTAL EXAMPLE 10

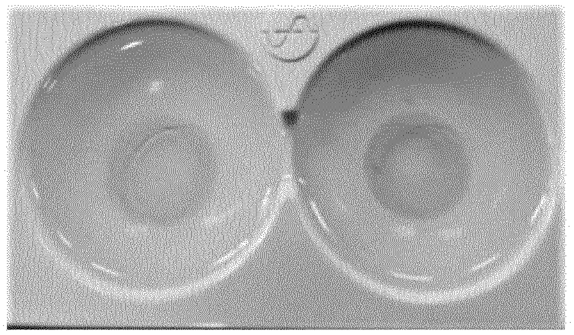

EXPERIMENTAL EXAMPLE 2 EXPERIMENTAL EXAMPLE 8

## INTERNATIONAL SEARCH REPORT

International application No. PCT/JP2019/025687

A. CLASSIFICATION OF SUBJECT MATTER
Int.Cl. G02C7/04(2006.01)i, A61F2/16(2006.01)i, G02B1/04(2006.01)i, G02B5/23(2006.01)i

According to International Patent Classification (IPC) or to both national classification and IPC

B. FIELDS SEARCHED

Minimum documentation searched (classification system followed by classification symbols)
Int.Cl. G02C7/04, A61F2/16, G02B1/04, G02B5/23

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-524857 A (TRANSITIONS OPTICAL, INC.) 30 August 2007, claims, paragraph [0014] (compound III), paragraphs [0028], [0030], [0064]-[0065], [0075]-[0078] & US 2004/0186241 A1 paragraphs [0017], [0040], [0044], [0101], [0112]-[0115], claims & WO 2004/086103 A1 & EP 1604231 A1 | 1-23<br>24-28 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 August 2019 (21.08.2019) | 03 September 2019 (03.09.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/025687

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2004/068215 A1 (MENICON CO., LTD.) 12 August 2004, p. 10, tables 1-2 & US 2005/0243273 A1 paragraph [0031], tables 1-2 & EP 1589367 A1 | 24-28 |
| Y | JP 2017-049615 A (JOHNSON & JOHNSON VISION CARE, INC.) 09 March 2017, paragraphs [0038]-[0042], fig. 1-6 & US 2011/0249234 A1 paragraphs [0042]-[0046], fig. 1-6 & WO 2011/130138 A1 & EP 2564264 A1 | 24-28 |
| A | JP 8-501504 A (BAUSCH & LOMB INCORPORATED) 20 February 1996, pp. 15-16 & US 5260000 A, columns 7-8 & WO 1994/003324 A1 & EP 652823 A1 | 1-28 |
| A | WO 2012/057096 A1 (MENICON CO., LTD.) 03 May 2012, paragraphs [0130]-[0169] & EP 2657304 A1 paragraphs [0130]-[0172] & CN 103210044 A | 1-28 |
| A | EP 0277639 A2 (TORAY INDUSTRIES, INC.) 10 August 1988, p. 20, example 25 & JP 1-258681 A & US 4929693 A & DE 3852831 C & AU 1118688 A & CA 1340939 C & AU 616650 B | 1-28 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004068215 A1
- JP 2017049615 A
- WO 200160881 A2
- WO 2005028465 A1
- JP 2011219513 A
- JP 2015503631 A
- JP 2006291006 A
- JP 2005181730 A
- WO 0070388 A1
- WO 2018021455 A1